(19) Europäisches Patentamt
European Patent Office
Office européen des brevets

(11) Veröffentlichungsnummer: **0 174 562**
**B1**

(12) **EUROPÄISCHE PATENTSCHRIFT**

(45) Veröffentlichungstag der Patentschrift: **09.01.91**

(21) Anmeldenummer: **85110917.3**

(22) Anmeldetag: **30.08.85**

(51) Int. Cl.$^5$: **C 07 D 249/10,** C 07 D 249/08, C 07 D 413/06, A 01 N 25/32, A 01 N 43/653, A 01 N 43/76, A 01 N 43/84, A 01 N 47/10, A 01 N 37/22, A 01 N 39/02, A 01 N 35/10 // C07C245/02, C07C241/02, C07C243/00

(54) **Pflanzenschützende Mittel auf Basis von 1,2,4-Triazolderivaten sowie neue Derivate des 1,2,4-Triazols.**

(30) Priorität: **11.09.84 DE 3433249**
**15.07.85 DE 3525205**

(43) Veröffentlichungstag der Anmeldung:
**19.03.86 Patentblatt 86/12**

(45) Bekanntmachung des Hinweises auf die Patenterteilung:
**09.01.91 Patentblatt 91/02**

(84) Benannte Vertragsstaaten:
**AT BE CH DE FR GB IT LI NL**

(56) Entgegenhaltungen:
**FR-A-2 526 271**
**US-A-4 115 095**

**BEILSTEINS "Handbuch Der Organischen Chemie", 4. Auflage, Band 26, 1937, Kapitel IVA: "Monocarbonsäuren", Verlag Julius Springer, Berlin;**

(73) Patentinhaber: **HOECHST AKTIENGESELLSCHAFT**
**Postfach 80 03 20**
**D-6230 Frankfurt am Main 80 (DE)**

(72) Erfinder: **Heubach, Günther, Dr.**
**Luisenstrasse 15**
**D-6233 Kelkheim (Taunus) (DE)**
Erfinder: **Bauer, Klaus, Dr.**
**Kolpingstrasse 7**
**D-6054 Rodgau (DE)**
Erfinder: **Bieringer, Hermann, Dr.**
**Eichenweg 26**
**D-6239 Eppstein/Taunus (DE)**

(56) Entgegenhaltungen:
**CHEMISCHE BERICHTE, Band 96, Nr. 12, 1963, Seiten 3120-3132, Verlag Chemie, Weinheim; M. REGITZ et al.: "Über die Bildung von 1.2.4-Triazolderivaten aus alpha-Acetamino-beta-dicarbonylverbindungen durch Japp-Klingemann-Spaltung mit Diazoniumsalzen"**

**TETRAHEDRON, Band 20, Nr. 2, 1964, Seiten 159-164, Nord Irland, GB; H. KANO et al.: "Isoxazoles-XV. Conversion of N-(5-methyl-3-isoxazolyl)-N'-arylformamidines into 1-aryl-3-acetonyl-1,2,4,-triazoles"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 174 562 B1

(56) Entgegenhaltungen:
JOURNAL OF THE CHEMICAL SOCIETY (C), Nr. 7, 1968, Seiten 824-830; E.J. BROWNE et al.: "Triazoles. Part IX.t 1,2,4-triazolyl ketones"

JOURNAL OF THE CHEMICAL SOCIETY. PERKIN TRANSACTIONS 1, 1977, Seiten 589-591; M. RUCCIA et al.: "Mononuclear heterocyclic rearrangements. Part 10. Rearrangements in the 1,2,5-oxadiazole series"

CHEMICAL ABSTRACTS, Band 86, Nr. 11, 14. März 1977, Seite 619, Spalte 2, Zusammenfassungsnr. 72658u, Columbus, Ohio, US; & JP - A - 51 88968

CHEMICAL ABSTRACTS, Band 54, Nr. 1, 10. Januar 1960, Spalten 510e-511a, Columbus, Ohio, US; N.N. VERESHCHAGINA et al.: "Synthesis of 3-acetyl-1,2,4-triazoles"

CHEMISCHE BERICHTE, Band 94, Nr. 7, 1961, Seiten 1868-1870; H. HELLMANN et al.: "Synthese von 1.2.4-Triazol-carbonsäure-(3)-estern"

CHEMISCHE BERICHTE, Band 95, Nr. 8, 1962, Seiten 1955-1957, Verlag Chemie, Weinheim; H. HELLMANN et al.: "1.2.4-Triazole, II. Synthese von 3-Cyan-1.2.4-triazolen"

CHEMISCHE BERICHTE, Band 98, Nr. 2, 1965, Seiten 642-649, Verlag Chemie, Weinheim; R. HUISGEN et al.: "1.3-Dipolare Cycloadditionen, XIII. Additionen der Nitrilimine an Oxime, Azine und andere CN-Doppelbindungen"

**Beschreibung**

Die vorliegende Erfindung betrifft pflanzenschützende Mittel, welche eine Triazolverbindung der Formel I

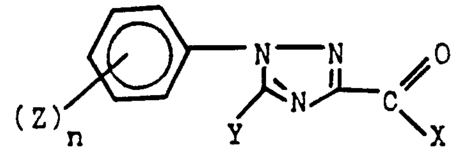 (I),

worin

Z bei gleicher oder verschiedener Bedeutung Halogen, Nitro, Cyano, Trifluormethyl, $(C_1—C_4)$-Alkyl, $(C_1—C_4)$Alkoxy, $(C_1—C_4)$Alkylthio, wobei die Alkyl-, Alkoxy- und Alkylthiogruppen durch ein oder mehrere Halogenatome, insbesondere Fluor oder Chlor, substituiert sein können, $(C_3—C_6)$Cycloalkyl, das durch $(C_1—C_4)$Alkyl substituiert sein kann, Phenyl oder Phenoxy, wobei Phenyl und Phenoxy durch Halogen ein- oder mehrfach und/oder einfach durch Trifluormethyl substituiert sein können,

Y Wasserstoff, $(C_1—C_4)$Alkyl, das ganz oder teilweise durch Halogenatome und/oder einfach durch $(C_1—C_4)$Alkoxy oder $(C_1—C_4)$Alkylthio substituiert sein kann, $(C_2—C_6)$Alkenyl, $(C_2—C_6)$Alkinyl, oder $(C_3—C_6)$Cycloalkyl, das durch $(C_1—C_4)$Alkyl und/oder einen Dichlorvinylrest substituiert sein kann,

X Hydroxy, $(C_1—C_4)$Alkyl, $(C_3—C_6)$Cycloalkoxy, Phenyl$(C_1—C_6)$alkoxy, Phenoxy, $(C_2—C_6)$Alkenyloxy, $(C_2—C_6)$Alkinyloxy, $(C_1—C_6)$Alkoxy, $(C_1—C_6)$Alkylthio, wobei die Alkoxy oder Alkylthiogruppe durch $(C_1—C_2)$Alkoxy-, Mono- oder Di-$(C_1—C_4)$Alkylaminocarbonyl, Phenylaminocarbonyl, N-$(C_1—C_4)$Alkyl-phenylaminocarbonyl, Mono- oder Di-$(C_1—C_6)$Alkylamino, $(C_1—C_6)$Alkyl-carbonyloxy, $(C_1—C_2)$Alkylthio, Cyano oder Halogen substituiert sein kann, einen Rest der Formeln

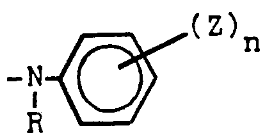 oder 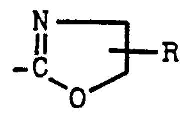 ,

worin jeweils R Wasserstoff oder $(C_1—C_4)$Alkyl bedeutet, Mono oder Di-$(C_1—C_4)$Alkylamino, $(C_5—C_6)$Cycloalkylamino, Piperidino, Morpholino oder 2,6-Dimethylmorpholino bedeuten, einen Rest der Formel

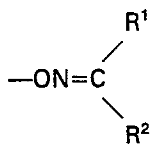

worin $R^1$ und $R^2$ gleich oder verschieden sein können und $(C_1—C_4)$Alkylreste bedeuten, worin $R^1$ und $R^2$ auch gemeinsam einen 5-, 6- oder 7-gliedrigen Cycloalkylrest bilden können, und

n die Zahl 0, 1, 2 oder 3 bedeuten, oder im Falle X = OH deren für die Landwirtschaft einsetzbare Salze enthalten.

Als Salze kommen beispielsweise Metallsalze wie Alkali- oder Erdalkalimetallsalze, insbesondere Natrium oder Kalium-Salze, Salze mit Ammonium, Mono-, Di-, Tri- oder Tetra-$(C_1—C_4)$alkylammonium oder mit Mono-, Di-, Tri oder Tetra-$(C_1—C_4)$alkanolammonium infrage.

Bevorzugt sind solche Verbindungen der Formel I, in denen

Y = $(C_1—C_2)$Alkyl, das ganz oder teilweise durch F, Cl oder Br substituiert sein kann,

Z = bei gleicher oder verschiedener Bedeutung Halogen, $(C_1—C_4)$Alkoxy, $(C_1—C_4)$Alkyl oder $CF_3$ und

X = $(C_1—C_6)$Alkoxy oder Hydroxy und

n = 1, 2 oder 3 bedeuten.

Von diesen Verbindungen sind besonders bevorzugt solche, bei denen Y = $CCl_3$, $CHCl_2$, $CHF_2CF_2$ oder $CH_3$ bedeuteten.

Diese Verbindungen der Formel I sind überraschenderweise geeignet, phytotoxische Nebenwirkungen von Pflanzenschutzmitteln bei Kulturpflanzen wirksam herabzusetzen oder ganz zu unterbinden. Solche Verbindungen werden auch als "Antidote" oder Safener bezeichnet.

Beilsteins Handb. der Org. Chem. 4, Aufl., Bd. 26 (1937), Seiten 284—287 beschreibt einige 1-Phenyl-5-alkyl-1,2,4-triazol-3-carbonsäuren und -ester. Aus Chem. Ber. 94, 1868 (1961), Chem. Ber. *96*, S. 3120—3132 (1963), Chem. Ber. *98*, 642 (1965), DE—1123331, Chem. Ber. *98*, 1955 (1962) und Chem. Abstr. *86*, CA86: 72658 u (1977) ist die Herstellung analoger, im Phenylrest gegebenenfalls substituierter Verbindungen sowie 1-Phenyl(ggf. subs.)-3-acetyl-1,2,4-triazole bekannt.

Tetrahedron *20*, S. 159—164 (1964) beschreibt die Herstellung von 1-Phenyl(ggf. subst.)-1,2,4-triazol-3-carbonsäuren. Aus Chem. Abstr. *54*, S. 510e—511a sind weitere 3-Acetyl-1,2,4-triazole bekannt.

Aus J. C. S. Perkin I *1977*, 589 und J. C. S. (C) *1968*, 824 sind N-Phenylamide von 1-Phenyl(ggf. subst.)-1,2,4-triazolen bekannt. Die genannte Literatur befaßt sich jedoch ausschließlich mit den Möglichkeiten zur

Herstellung der Verbindungen. Die Safener-Wirkungen der Verbindungen waren jedoch nicht erkannt worden.

Aus der US—A—4,115,095 sind noch 5-Aryl-1,2,4-thiadiazol-3-carbonsäure(ester) bekannt, die als Safener in Getreide oder als den Zuckergehalt in Zuckerrohr steigernde Mittel eingesetzt werden können.

Gegenstand der vorliegenden Erfindung sind auch die Verbindungen der obengenannten Formel (I), die neu, also nicht vorbeschrieben sind. Es sind dies die Verbindungen der Formel (I), ausgenommen solche, in welchen

a) y = H, $(Z)_n$ = H und X = OH, $OCH_3$, $OC_2H_5$ oder $NHC_6H_5$
b) y = H, $(Z)_n$ = 4-$OCH_3$ und X = OH, $OCH_3$ oder $NHC_6H_5$,
c) y = H, $(Z)_n$ = 3-$OCH_3$, 4-Cl, 4-$CH_3$ und X = $OCH_3$,
d) y = H, $(Z)_n$ = 3-Cl und X = OH,
e) y = $CH_3$, $(Z)_n$ = H oder $OCH_3$ und X = OH,
f) y = $CH_3$, $(Z)_n$ = 4-$NO_2$ und X = OH, $OCH_3$ oder $OC_2H_5$,
g) y = $CH_3$, $(Z)_n$ = 2-Cl, 4-$NO_2$—2—$CH_3$ oder 4-$NO_2$—2—$OCH_3$ und X = $OC_2H_5$,
h) y = $C_2H_5$, $(Z)_n$ = H und X = OH
i) y = n-$C_3H_7$ oder i-$C_3H_7$, $(Z)_n$ = H und X = OH, $OCH_3$ oder $OC_2H_5$
k) x = $CH_3$, y = H oder $C_2H_5$ und $(Z)_n$ = H, 4-Cl oder 4-$OC_2H_5$ oder
l) x = $CH_3$, y = $CH_3$ und $(Z)_n$ = H, 4-Cl, 4-$OCH_3$, 4-$OC_2H_5$ oder 4-$NO_2$

bedeuten.

Gegenstand der vorliegenden Erfindung ist auch ein Verfahren zur Herstellung der neuen Verbindungen der Formel I und deren Salze, dadurch gekennzeichnet, daß man

a) eine Verbindung der Formel II

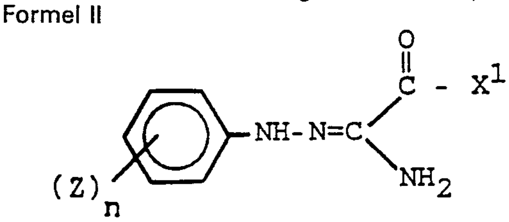

(II),

worin $X^1$ die Bedeutung von X außer Hydroxy besitzt,

$a_1$) mit einer Verbindung der Formel Y—CO—Cl oder
$a_2$) mit einem Säureanhydrid der Formel Y—CO—O—CO—Y oder
$a_3$) mit einem Orthoester der Formel Y—C$(OR^1)_3$, worin $R^1$ = $(C_1—C_4)$Alkyl bedeutet, oder

b) für Verbindungen mit Y = H, $(C_1—C_4)$Alkyl eine Verbindung der Formel III

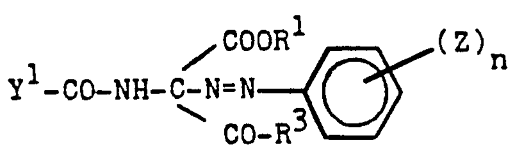

(III),

worin $Y^1$ = H oder $(C_1—C_4)$Alkyl und $R^3$ = $(C_1—C_4)$Alkoxy oder $CH_3$ bedeuten, mit einer Base umsetzt, wobei die unter $a_1$) und $a_2$) erhaltenen Verbindungen gegebenenfalls in Essigsäure erhitzt werden, und die erhaltenen Verbindungen der Formel I gegebenenfalls durch Derivatisierung in andere Verbindungen der Formel I oder deren Salze überführt werden.

Überraschend bei den Verfahrensvarianten $a_1$) und $a_2$) ist, daß neben der Addition des Säurechlorids oder -anhydrids an die freie Aminogruppe unmittelbar nachfolgend in einer Zweistufenreaktion häufig die direkte Cyclisierung zu den Verbindungen der Formel I beobachtet wird. Ferner ist überraschend, daß im Falle von $a_1$) die Reaktion ohne Basenzusatz erfolgt. Im Gegenteil führt der sonst übliche Zusatz von Basen zu harzigen Produkten.

Die Umsetzungen der Verbindungen der Formel II mit einem Carbonsäurechlorid (YCOCl), Säureanhydrid oder Orthoester werden zweckmäßigerweise in einem organischen inerten protonenfreien Lösungsmittel durchgeführt. Im Falle des Säureanhydrids oder Orthoesters (Varinate $a_2$ oder $a_3$) kann das betreffende Reagens auch selbst als Lösungsmittel dienen. Die Verfahrensvariante $a_3$) läßt sich vorteilhaft in Gegenwart eines sauren Katalysators, insbesondere einer organischen Säure wie p-Toluolsulfonsäure, durchführen.

Als inerte Lösungsmittel für die Verfahrensvarinaten $a_1$), $a_2$) und $a_3$) eignen sich insbesondere Aromaten wie Benzol, Toluol, Xylol, Chlorbenzol oder cyclische Ether-Verbindungen wie Tetrahydrofuran oder Dioxan oder auch Ketone wie Aceton und dipolar aprotische Lösungsmittel wie Dimethylformamid. Die Reaktionstemperaturen variieren je nach Lösungsmittel zwischen 10°C und dem Siedepunkt des Reaktionsgemisches. Bei Einsatz von aromatischen Lösungsmitteln im Falle von Varinate $a_1$) wird nach Zugabe des Carbonsäurechlorids das entstehende Wasser unter Rückfluß mittels eines Wasserabscheiders entfernt. Verschiedentlich entsteht im Falle der Verfahrensvarianten $a_1$) oder $a_2$) in Abhängigkeit der Rest $(Z)_n$ und X der Verbindungen der Formel II zunächst ein Zwischenprodukt der Formel IV,

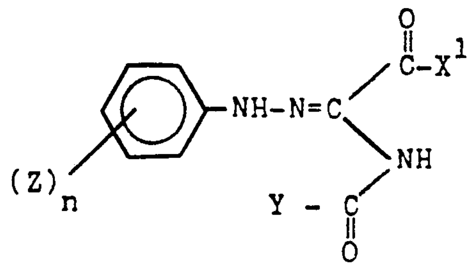

(IV)

welches unter Umständen isoliert werden kann. Wenn die Reaktion auf dieser Stufe bei Verwendung der obengenannten Lösungsmittel stehen bleibt, muß eine Nachreaktion in Essigsäure durchgeführt werden. Hierzu wird das Zwischenprodukt der Formel IV in Essigsäure zwischen ca. 50°C und Rückflußtemperatur erhitzt. Diese Nachreaktion kann im Eintopfverfahren durchgeführt werden, wobei vor Zugabe der Essigsäure das organische Lösungsmittel der ersten Verfahrensstufe abdestilliert wird.

Die Verfahrensvariante b) zur Herstellung der Verbindungen der Formel I ist im Prinzip gemäß Chem. Ber. *96* 3120 (1963) bekannt. Als Basen werden insbesondere anorganische Basen, bevorzugt Natrium- oder Kaliumhydroxid, eingesetzt. Die Herstellung der Verbindungen der Formel III erfolgt, wie dort beschrieben, durch Reaktion von Acetamidomalonester oder Acetaminoacetessigester-Verbindungen mit Diazoniumsalzen.

Die so erhaltenen Verbindungen der Formel I können durch übliche Derivatisierungsreaktionen in andere Verbindungen der Formel I überführt werden. So können die Verbindungen der Formel I mit X = OH aus den Ester-Verbindungen der Formel I durch saure oder alkalische Hydrolyse gewonnen werden. Aus den Säuren der Formel I (X = OH) können auf üblichem Wege durch Zugabe entsprechender Basen die Salze der Verbindungen der Formel I erhalten werden. Ferner können aus den Esterverbindungen der Formel I in üblicher Weise andere Ester oder Amide der Formel I gewonnen werden, beispielsweise über die entsprechenden Säurechloride.

Die Herstellung von Verbindungen der Formel II ist im Prinzip bekannt; die Verbindungen der Formel II lassen sich durch Umsetzung von α-Chlorhydrazonen der Formel V

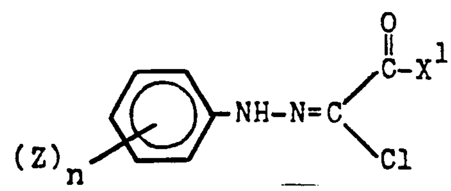

(V)

mit Ammoniak herstellen. Die Verbindungen der Formel V wiederum sind durch Umsetzung von Phenyldiazoniumsalzen mit α-Halogenacetessigestern bzw. α-Halogen-β-diketonen zugänglich. Beide Reaktionen sind in J. Chem. Soc. *87* 1859 (1905) und Ber. d. dt. Chem. Ges. *50* 1482 (1917) beschrieben.

Die Verbindungen der Formel I eignen sich in erster Linie zum Schutz von Kulturpflanzen vor toxischen Nebenwirkungen von Herbiziden.

Die Verbindungen der Formel I können zusammen mit anderen Herbiziden ausgebracht werden und sind dann in der Lage, schädliche Nebenwirkungen dieser Herbizide zu antagonisieren oder völlig aufzuheben, ohne die herbizide Wirksamkeit dieser Herbizide gegen Schadpflanzen zu beeinträchtigen. Hierdurch kann das Einsatzgebiet herkömmlicher Pflanzenschutzmittel ganz erheblich vergrößert werden.

Safener für Herbizide vom Typ der Phenoxyphenoxycarbonsäureester sind aus der veröffentlichten Europäischen Patentanmeldung 31 938 bekannt. Diese Safener besitzen jedoch unzureichende Wirkungen.

Herbizide, deren phytotoxische Nebenwirkungen mittels der Verbindungen der Formel I herabgesetzt werden können, sind z.B. Carbamate, Thiolcarbamate, Halogenacetanilide, substituierte Phenoxy-, Naphthoxy- und Phenoxyphenoxycarbonsäurederivate sowie Heteroaryloxyphenoxycarbonsäurederivate wie Chinolyloxy-, Chinoxalyloxy-, Pyridyloxy-, Benzoxazolyloxy-, Benzthiazolyloxy-phenoxy-carbonsäureester und ferner Dimedonoximabkömmlinge. Bevorzugt hiervon sind Phenoxyphenoxy- und Heteroaryloxyphenoxycarbonsäureester. Als Ester kommen hierbei insbesondere niedere Alkyl-, Alkenyl und Alkinylester in Frage.

Beispielsweise seien, ohne daß dadurch eine Beschränkung erfolgen soll, folgende Herbizide genannt:

A) Herbizide vom Typ der Phenoxyphenoxy- und Heteroaryloxyphenoxycarbonsäure-($C_1$—$C_4$)alkyl-, ($C_2$—$C_4$)alkenyl- und ($C_3$—$C_4$)alkinylester wie

2-(4-(2,4-Dichlorphenoxy)-phenoxy)-propionsäuremethylester,
2-(4-(4-Brom-2-chlorphenoxy)-phenoxy)-propionsäuremethylester,
2-(4-(4-Trifluormethylphenoxy)-phenoxy)-propionsäuremethylester,
2-(4-(2-Chlor-4-trifluormethylphenoxy)-phenoxy)-propionsäuremethylester,
2-(4-(2,4-Dichlorbenzyl)-phenoxy)-propionsäuremethylester,
4-(4-(4-Trifluormethylphenoxy)-phenoxy)-pent-2-en-säureethylester,
2-(4-(3,5-Dichlorpyridyl-2-oxy)-phenoxy)-propionsäureethylester,

2-(4-(3,5-Dichlorpyridyl-2-oxy)-phenoxy)-propionsäure-propargylester,
2-(4-(6-Chlorbenzoxazol-2-yl-oxy)-phenoxy)-propionsäureethylester,
2-(4-(6-Chlorbenzthiazol-2-yl-oxy)-phenoxy)-propionsäureethylester,
2-(4-(3-Chlor-5-trifluormethyl-2-pyridyloxy)-phenoxy)-propionsäuremethylester,
2-(4-(5-Trifluormethyl-2-pyridyloxy)-phenoxy)-propionsäurebutylester,
2-(4-(6-Chlor-2-chinoxalyloxy)-phenoxy)-propionsäureethylester,
2-(4-(6-Fluor-2-chinoxalyloxy)-phenoxy)-propionsäureethylester,
2-(4-(6-Chlor-2-chinolyloxy)-phenoxy)-propionsäureethylester,
B) Chloracetanilid-Herbizide wie
N-Methoxymethyl-2,6-diethyl-chloracetanilid,
N-(3'-Methoxyprop-2'-yl)-methyl-6-ethyl-chloracetanilid,
N-(3-Methyl-1,2,4-oxdiazol-5-yl-methyl)-chloressigsäure-2,6-dimethylanilid,
C) Thiocarbamate wie
S-Ethyl-N,N-dipropylthiocarbamat oder
S-Ethyl-N,N-diisobutylthiocarbamat
D) Dimedon-Derivate wie
2-(N-Ethoxybutyrimidoyl)-5-(2-ethylthiopropyl)-3-hydroxy-2-cyclohexen-1-on,
2-(N-Ethoxybutyrimidoyl)-5-(2-phenylthiopropyl)-3-hydroxy-2-cyclohexen-1-on oder
2-(1-Allyloxyiminobutyl)-4-methoxycarbonyl-5,5-dimethyl-3-oxocyclohexenol.

Das Mengenverhältnis Safener:Herbizid kann innerhalb weiter Grenzen, im Bereich zwischen 1:10 und 10:1, insbesondere zwischen 2:1 und 1:10, schwanken. Die jeweils optimalen Mengen an Herbizid und Safener sind abhängig vom Typ des verwendeten Herbizids oder vom verwendeten Safener sowie von der Art des zu behandelnden Pflanzenbestandes und lassen sich von Fall zu Fall durch entsprechende Versuche ermitteln.

Haupteinsatzgebiete für die Anwendung der Safener wird vor allem Getreidekulturen (Weizen, Roggen, Gerste, Hafer), Reis, Mais, Sorghum, aber auch Baumwolle, Zuckerrüben, Zukkerrohr und Sojabohne.

Die Safener der Formel I können je nach ihren Eigenschaften zur Vorbehandlung des Saatgutes der Kulturpflanze (Beizung der Samen) verwendet werden oder vor der Saat in die Saatfurchen eingebracht werden oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein. Bevorzugt ist die gemeinsame Anwendung mit dem Herbizid. Hierzu können Tankmischungen oder Fertigformulierung eingesetzt werden.

Gegenstand der vorliegenden Erfindung ist daher auch ein Verfahren zum Schutz von Kulturpflanzen vor phytotoxischen Nebenwirkung von Herbiziden, das dadurch gekennzeichnet ist, daß eine wirksame Menge einer Verbindung der Formel I vor, nach oder gleichzeitig mit dem Herbizid appliziert wird.

Die Verbindungen der Formel I weisen außerdem fungizide Wirkungen auf und lassen sich daher zur Bekämpfung von phytopathogenen Pilzen wie beispielsweise echten Mehltaupilzen, Rostpilzen, Pilzen der Gattung Phytopthora, Botrytis, Piricularia Venturia inequalis, einsetzen. Für die Anwendung können die Verbindungen der Formel I mit üblichen Formulierungshilfmitteln zu Stäubemitteln, Spritzpulvern, Dispersionen, Emulsionskonzentraten usw. zubereitet werden, die entweder als solche angewendet werden (Stäubemittel, Pellets) oder vor der Anwendung in einem Lösungsmittel (Wasser) gelöst oder dispergiert werden.

Darüber hinaus besitzen die Verbindungen der Formel I zum Teil pflanzenwachstumsregulierende Wirkungen. Sie greifen regulierend in den pflanzeneigenen Stoffwechsel ein und können daher zur gezielten Beeinflussing von Pflanzeninhaltsstoffen sowie zur Ernteerleichterung wie zum Auslösen von Desikkation und Wuchsstauchung eingesetzt werden.

Die erfindungsgemäßen Mittel können als Spritzpulver, emulgierbare Konzentrate, versprühbare Lösungen, Stäubemittel, Beizmittel, Granulate oder Mikrogranulate in den üblichen Zubereitungen angewendet werden.

Unter Spritzpulvern werden in Wasser gleichmäßig dispergierbare Präparate verstanden, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Ihre Herstellung erfolgt in üblicher Weise z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil ganz oder auch teilweise entfallen. Als Emulgatoren können beispielsweise verwendet werden: Alkylarylsulfonsaure Calciumsalze wie Cadodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel werden durch Vermahlen des Wirkstoffes mit fein verteilten, festen Stoffen, z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise — gewünschtenfalls in Mischung mit Düngemitteln — granuliert werden.

In Spritzpulvern beträgt die Wirkstoffkonzentration etwa 10 bis 90 Gew.-%; der Rest zu 100 Gew.-% besteht aus üblichen Formulierungsbestandteilen. Bei emulgierbaren Konzentration kann die Wirkstoffkonzentration etwa 10 bis 80 Gew.-% betragen. Staubfähige Formulierungen enthalten meistens 5 bis 20 Gew.-% an Wirkstoff, versprühbare Lösungen etwa 1 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Die benötigten Aufwandmengen der Verbindungen der Formel I beim Einsatz als Safener können je nach Indikation und verwendetem Herbizid innerhalb weiter Grenzen schwanken und variieren im allgemeinen zwichen 0,01 und 10 kg Wirkstoff/ha.

Folgende Beispiele dienen zur Erläuterung der Erfindung.

A. Formulierungsbeispiele

a) Ein Stäubemittel wird erhalten, indem man 10 Gew.-Teile einer Verbindung der Formel I und 90 Gewichtsteile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel I, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel I mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z.B. ca. 255 bis über 377°C) mischt und in einer Reibkugelmühle auf eine Feinheit von unter 5 Mikron vermahlt.

d) Ein emulgierbares Konzentrat wird erhalten aus 15 Gewichtsteilen einer Verbindung der Formel I, 75 Gewichtsteilen Cyclohexanon als Lösungsmittel und 10 Gewichtsteilen oxethyliertes Nonylphenol als Emulgator.

e) Ein Wasser leicht emulgierbares Konzentrat aus einem Phenoxycarbonsäureester und einem Antidot (10:1) wird erhalten aus

12,00 Gew.-% 2-[4-(6-Chlorbenzoxazol-2-yl-oxy)-phenoxy]propionsäureethylester
1,20 Gew.-% Verbindung der Formel I
69,00 Gew.-% Xylol
7,80 Gew.-% dodecylbenzolsulfonsaurem Calcium
6,00 Gew.-% ethoxyliertem Nonylphenol (10 EO)
4,00 Gew.-% ethoxyliertem Rizinusöl (40 EO)

Die Zubereitung erfolgt wie unter Beispiel a) angegeben.

f) Ein in Wasser leicht emulgierbares Konzentrat aus einem Phenoxycarbonsäureester und einem Antidot (1:10) wird erhalten aus

4,0 Gew.-% 2-[4-(6-Chlorbenzoxazol-2-yl-oxy)-phenoxy]propionsäureethylester
40,0 Gew.-% Verbindung der Formel I
30,0 Gew.-% Xylol
20,0 Gew.-% Cyclohexanon
4,0 Gew.-% dodecylbenzolsulfonsaurem Calcium
2,0 Gew.-% ethoxyliertem Rizinusöl (40 EO)

B. Chemische Beispiele

*Vorprodukte*

1) α-Chlor-α-(3-trifluormethylphenylhydrazono)-glyoxylsäuremethylester

161,1 (1,0 Mol) 3-Trifluormethylanilin wurden in einer Mischung aus 400 ml Wasser/326 ml HCl konz. gelöst und bei 0°C 70 g Natriumnitrit in 400 ml Wasser unter kräftigem Rühren zugetropft.

Die resultierende Diazoniumsalzlösung wurde anschließend unter kräftigem Rühren in ein auf +10°C

gehaltenes Gemisch aus 165,5 g α-Chloracetessigsäuremethylester, 800 ml Wasser, 444 g Natriumacetat und 1000 ml Ethanol getropft. Nach 3-stündigem Nachrühren wurde mit Wasser verdünnt, abgesaugt und das Rohprodukt in Methanol ausgekocht.

*Ausbeute*: 263,6 g = 94%
Schmp.: 145°C

2) α-Amino-α(3-trifluormethylphenylhydrazono)-glyoxylsäuremethylester

100 g (0,356 Mol) α-Chlor-α-(3-trifluormethylphenylhydrazono)-glyoxylsäuremethylester wurden in 560 ml Tetrahydrofuran gelöst und bei +15 bis +20°C tropfenweise mit 61 g 25%igen wässrigem Ammoniak versetzt. Nach 5-stündigem Nachrühren bei Raumtemperatur wurde in Wasser gegossen, abgesaugt und der Rückstand in Methanol ausgekocht.

*Ausbeute*: 88,3 g = 95%
Schmp.: 138°C

*Endprodukte*

3) 1-(3-Trifluormethylphenyl)-3-methoxy-carbonyl-5-trichlormethyl-1,2,4-triazol

a) *in Toluol als Lösungsmittel*

26,1 g (0,1 Mol) α-Amino-α-(3-trifluormethylphenylhydrazono)-glyoxylsäuremethylester wurden in 150 ml Toluol vorgelegt, 0,12 Mol Trichloracetylchlorid unter Rühren zugetropft und anschließend unter Rückfluß so lange am Wasserabscheider gekocht, bis kein Wasser mehr übergeht (ca. 1 Std.).

Nach Abkühlen wurde die Toluollösung mit Wasser gewaschen und das Toluol im Vakuum abdestilliert. Das zurückbleibende Rohprodukt wurde aus Methanol umkristallisiert.

*Ausbeute*: 24 g = 61,7%
Hellgelbe Kristalle vom Schmp.: 92—93°C.

b) *in Tetrahydrofuran als Lösungsmittel*

182,8 g (0,7 Mol) α-Amino-α-(3-trifluormethylphenylhydrazono)-glyoxylsäuremethylester wurden in 1200 ml Tetrahydrofuran gelöst und ohne zu kühlen in 10 Minuten 191 g (1,05 Mol) Trichloracetylchlorid unter Rühren zugetropft. Es wurde 30 Minuten bei Raumtemperatur gerührt, anschließend 15 Minuten unter Rückfluß gehalten und 5 Stunden bei Raumtemperatur nachgerührt. Nach Eingießen in Wasser wurde von ausgefallenen Kristallen abgesaugt und mit Wasser gewaschen. Gelbliche Kristalle.

*Ausbeute*: 233,8 g = 86%
Schmp.: 90°C

Nach Umkristallisieren aus Methanol schmilzt eine Probe bei 92—93°C.

4) 1-(3-Trifluormethylphenyl)-5-trichlormethyl-1,2,4-triazol-3-carbonsäure

210 g (0,54 Mol) 1-(3-Trifluormethylphenyl)-3-methoxycarbonyl-5-trichlormethyl-1,2,4-triazol wurden in 540 ml Methanol vorgelegt und mit 0,57 Mol NaOH (22,8 g) in 100 ml Wasser versetzt. Nach 5-stündigem Rühren bei Raumtemperatur wurde in 4000 ml Wasser gegossen, von ungelöster Substanz filtriert und das klare Filtrat mit Salzsäure auf pH 1 gestellt. Farblose Kristalle fielen aus, die abgesaugt und mit Wasser gewaschen wurden. Die in Wasser ungelöst gebliebene Substanz — das Natriumsalz der Carbonsäure — wurde in einer Mischung aus 2000 ml Methanol/1000 ml Wasser gelöst, mit Salzsäure auf pH 1 gestellt, der farblose kristalline Niederschlag abgesaugt und mit Wasser gewaschen.

Die vereinten Fraktionen der Carbonsäure wurden aus 1000 ml Toluol umkristallisiert.

*Ausbeute*: 174 g = 84%
Schmp.: 133—136°C

5) 1-(3-Trifluormethylphenyl)-3-isopropoxy-carbonyl-5-trichlormethyl-1,2,4-triazol

20 g (0,0534 Mol) 1-(3-Trifluormethylphenyl)-5-trichlormethyl-1,2,4-triazol-3-carbonsäure wurden in 70 ml Thionylchlorid 30 Minuten unter Rückfluß gehalten, das überschüssige Thionylchlorid in Vakuum entfernt und das rohe Carbonsäurechlorid in 120 ml Isopropanol 60 Minuten gekocht. Nach Erkalten der Lösung wurde in Eiswasser gegossen und von farblosen Kristallen abgesaugt.

Die rohen, farblosen Kristalle wurden bei Raumtemperatur in einer Mischung aus 50 ml Methanol/Wasser = 1/2 verrührt, erneut abgesaugt und an der Luft getrocknet.

*Ausbeute*: 19,4 g = 87,3%
Schmp.: 91°C

6) 1-(3-Trifluormethylphenyl)-5-trichlormethyl-1,2,4-triazol-3-carbonsäure-3'-trifluormethylanilid

0,0345 Mol Carbonsäure wurden analog Beispiel 5.) in das Säurechlorid überführt, dieses in 80 ml Toluol gelöst und bei +5°C in 20 Minuten tropfenweise mit einer Mischung aus 0,0345 Mol 3-Trifluormethylanilin (5,56 g) und 0,0345 Mol Triethylamin (3,5 g) versetzt. Nach 5-stündigem Rühren bei Raumtemperatur wurde mit Wasser gewaschen und das Toluol im Vakuum entfernt. Der Rückstand wurde aus 40 ml Methanol umkristallisiert. Farblose Kristalle von Schmp. 126°C.

*Ausbeute*: 14,4 g = 81%

7) 1-(3-Trifluormethylphenyl)-3-methoxy-carbonyl-5-dichlormethyl-1,2,4-triazol

0,1 Mol (26,1 g) α-Amino-α-(3-trifluormethylphenylhydrazono)-glyoxylsäure-methylester wurden in 150 ml Toluol gelöst, mit 0,105 Mol (15,5 g) Dichloracetylchlorid bei Raumtemperatur tropfenweise unter Rühren versetzt und 60 Minuten am Wasserabscheider unter Rückfluß gehalten. Nach Erkalten wurde mit Wasser mehrfach gewaschen und das Toluol im Vakuum entfernt. Es blieb ein hell-honigfarbener Sirup zurück. $n_D^{30}$: 1,5259.

*Ausbeute*: 28,2 g = 80%

NMR-Spektrum in $CDCl_3$: *$COOCH_3$* δ 4,05; *$CHCl_2$* δ 6,72 RF-Wert in Toluol/Ethylacetat 2/1: 0,52.

8) 1-(2,6-Diethylphenyl)-3-methoxycarbonyl-5-trichlormethyl-1,2,4-triazol

0,15 Mol (37,4 g) α-Amino-α-(2,6-diethylphenylhydrazono)-glyoxylsäuremethylester wurden in 165 ml Tetrahydrofuran gelöst und mit 0,194 Mol (35,4 g) Trichloracetylchlorid versetzt. Nach 3-stündigem Rühren bei Raumtemperatur wurde in Wasser gegossen, mit Methylenchlorid ausgerührt, die organische Phase mit Wasser gewaschen und das Lösungsmittel im Vakuum entfernt.

*Ausbeute*: 51,7 g = 91,5%

Hellbrauner Sirup, bei dem es sich laut NMR-Spektrum und Elementaranalyse um die offenkettige Verbindung

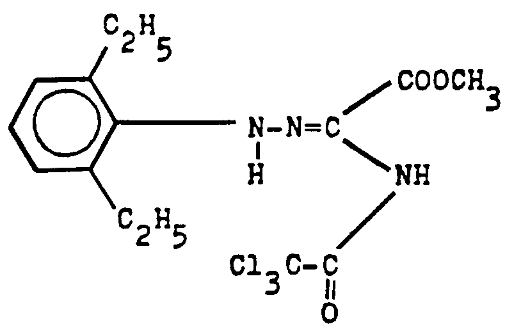

handelt.

18,9 g (0,047 Mol) dieser Verbindung wurden in 120 ml Eisessig 1,5 Stunden unter Rückfluß gehalten, nach Abkühlen in Wasser gegossen und mit Methylenchlorid ausgerührt. Die organische Phase wurde dreimal mit Wasser gewaschen und das Lösungsmittel im Vakuum entfernt.

Die zurückbleibenden hellgelben Kristalle wurden mit 20 ml Methanol verrührt und abgesaugt.

*Ausbeute*: 16,4 g = 91%
Schmp.: 76—77°C

9) 1-(3-Trifluormethylphenyl)-3-methoxycarbonyl-5(2,2-dimethylethen-1-yl)-1,2,4-triazol

26.1 g (0.1 Mol) α-Amino-α-(3-trifluormethylphenylhydrazono)glyoxylsäuremethylester wurden in 150 ml Tetrahydrofuran gelöst und ohne Kühlung in 10 Minuten 15,4 g (0.13 Mol) β,β-Dimethylacrylsäurechlorid zugetropft. Nach einstündigem Kochen unter Rückflußbedingungen wurde der größte Teil des Tetrahydrofurans bei Normaldruck abdestilliert und das Reaktionsgemisch mit überschüssigem Wasser verrührt. Nach Dekantieren des Wassers wurden die rohen Kristalle aus Methanol umkristallisiert.

*Ausbeute*: 24 g = 73.7%
Schmp: 143—144°C

10) 1-(4-Fluorphenyl)-3-methoxycarbonyl)-5-methoxymethyl-1,2,4-triazol

21,1 g (0,1 Mol) α-Amino-α-(4-fluorphenylhydrazono)-glyoxylsäuremethylester wurden in 140 ml Toluol gelöst und ohne Kühlung in 10 Minuten 14.1 g (0.13 Mol) Methoxyacetylchlorid zugetropft. Es wurde 1,5 Stunden am Wasserabscheider gekocht, die erkaltete Toluollösung zweimal mit je 200 ml Wasser gewaschen, das Toluol im Vakuum abdestilliert und der Rückstand aus Methanol umkristallisiert.

*Ausbeute*: 15,2 g = 57,4%
Schmp: 93—94°C

11) 1-(2,4-Dichlorphenyl)-3-ethoxy-carbonyl-5-methyl-1,2,4-triazol

11a) α-*(2,4-Dichlorphenylazo)-acetamino-malonsäurediethylester*

0,2 Mol (33,4 g) 2,4-Dichloranilin wurden in 60 ml Wasser und 75 ml konz. Salzsäure kurz aufgekocht, die Suspension auf +5°C gekühlt und bei dieser Temperatur mit 0.2 Mol (13.8 g) Natriumnitrit in 25 ml Wasser diazotiert.

Die Lösung des Diazoniumsalzes wurde in 15 Min. bei +5 bis 7°C zu einer kräftig gerührten Mischung aus 300 ml Ethanol, 200 ml Wasser, 100 g Natriumacetat und 0.2 Mol (43.3 g) Acetaminomalonesäurediethylester getropft. Nach einstündigem Nachrühren bei Raumtemperatur wurde in Wasser gegossen, abgesaugt und die Kristalle mit Wasser gewaschen. Nach Umkristallisieren aus Ethanol/Wasser schmelzen die Kristalle bei 123—124°C.

*Ausbeute*: 71 g = 91%

11b) *1-(2,4-Dichlorphenyl)-5-methyl-1,2,4-triazol-3-carbonsäure*

39 g (0.1 Mol) des Produktes aus Beispiel 11a wurden in einer Mischung aus 165 ml Wasser und 24.2 g Kaliumhydroxyd 5 Min. unter Rückfluß gekocht. Die resultierende klare Lösung wurde nach Abkühlen auf 60°C mit konz. Salzsäure angesäuert, die ausgefallene 1-(2,4-Dichlorphenyl)-5-methyl-1,2,4-triazol-3-carbonsäure abgesaugt und mit Wasser neutral gewaschen.

*Ausbeute*: 25.6 g = 94%
Schmp: 163—164°C

11c) *1-(2,4-Dichlorphenyl)-3-ethoxycarbonyl-5-methyl-1,2,4-triazol*

0.10 Mol 11b) wurde 2 Stunden in 150 ml Thionylchlorid am Rückfluß gekocht, das überschüssige Thionylchlorid im Vakuum entfernt und das rohe Säurechlorid in 500 ml Ethanol 30 Min. zum Sieden erhitzt. Nach Eingießen in Wasser wurden die ausgefallenen Kristalle abgesaugt und mit Wasser neutral gewaschen.

*Ausbeute*: 24 g = 80%
Schmp: 131—132°C

Nach Umkristallisieren aus Methanol schmelzen die Kristalle bei 133—134°C.

12) 1-(2,4-Dichlorphenyl)-3-ethoxycarbonyl-5-methyl-1,2,4-triazol

12a) 0.1 Mol α-Amino-α-(2,4-dichlorphenylhydrazono)glyoxylsäureethylester (27,6 g) wurden in 150 ml Acetanhydrid 2 Stunden unter Rückfluß gehalten, das überschüssige Acetanhydrid in Vakuum entfernt und das Rohprodukt aus Ethanol umkristallisiert.

*Ausbeute*: 21.6 g = 72%
Schmp: 133—134°C

12b) 0.1 Mol α-Amino-α-(2,4-dichlorphenylhydrazono)glyoxylsäurethylester (27.6 g) wurden in 130 ml ortho-Essigsäuretriethylester 4 Stunden unter Rückfluß gehalten, der überschüssige Orthoester im Vakuum entfernt und das Produkt aus Ethanol umkristallisiert.

*Ausbeute*: 18.9 g = 63%
Schmp: 133—134°C

13) 1-(2,4-Dichlorphenyl)-3-ethoxycarbonyl-1,2,4-triazol

0.1 Mol α-Amino-α-(2,4-dichlorphenylhydrazono)glyoxylsäureethylester (27.6 g) wurden in 120 ml ortho-Ameisensäuretrimethylester 5 Stunden unter Rückfluß gehalten, der überschüssige Orthoester in Vakuum entfernt und der Rückstand aus Ethanol umkristallisiert.

*Ausbeute*: 21.4 g = 75%
Schmp: 105—106°C

14) 1-Phenyl-3-acetyl-5-trichlormethyl-1,2,4-triazol

0.1 Mol α-Amino-α-phenylhydrazonomethylglyoxal (17.7 g) wurden in 150 ml Tetrahydrofuran auf einmal unter Rühren mit 23.6 g (0.13 Mol) Trichloracetylchlorid versetzt. Nach einstündigem Kochen unter Rückfluß wurde in 1 l Wasser gegossen, das Wasser vom ausgefallenen Rohprodukt dekantiert und aus Methanol umkristallisiert.

*Ausbeute*: 20.1 g = 66%
Schmp: 139—140°C

Analog den Herstellungsbeispielen 3 bis 14 wurden die in Tabelle I aufgeführten Verbindungen der Formel I hergestellt.

Tabelle I

| Bsp. Nr. | $(Z)_n$ | Y | X | Schmp. (°C) $n_D^{30}$ | Herst. gem. Beisp. Nr. |
|---|---|---|---|---|---|
| 15 | 3-Cl | $CCl_3$ | $-OC_2H_5$ | 76-77 | 3a |
| 16 | 3-Cl | $CCl_3$ | -OH | 124-127 | 4 |
| 17 | 3-Cl | $CHCl_2$ | $-OC_2H_5$ | Sirup | 3a |
| 18 | 2-Cl | $CCl_3$ | $-OC_2H_5$ | 99-100 | 3a |
| 19 | 2-Cl | $CCl_3$ | -OH | 204-205 | 4 |
| 20 | 2-Cl | $CCl_3$ | $-OCH_3$ | 114-115 | 3b |
| 21 | 2-Cl | $CCl_3$ | $-OCH_2CH_2CH_3$ | 90-92 | 5 |
| 22 | 4-Cl | $CHCl_2$ | $-OCH_3$ | 152-153 | 3a |
| 23 | $3,5-Cl_2$ | $CHCl_2$ | $-OCH_3$ | 1,5809 | 3a |
| 24 | $4-NO_2$ | $CHCl_2$ | $-OCH_3$ | 149-150 | 3a |
| 25 | $3,5-Cl_2$ | $CCl_3$ | $-OCH_3$ | 143-145 | 3b |
| 26 | $3,5-Cl_2$ | $CCl_3$ | -OH | 194-195 | 4 |
| 27 | 2-Cl | $CHCl_2$ | $-OC_2H_5$ | 135-136 | 3a |
| 28 | 3-Cl | $CHCl_2$ | -OH | 114 | 4 |
| 29 | 2-Cl | $CHCl_2$ | $-OCH_3$ | 158 | 3a |
| 30 | $4-NO_2$ | $CCl_3$ | $-OCH_3$ | 201-203 | 3b |
| 31 | H | $CCl_3$ | $-OCH_3$ | 103-104 | 3b |
| 32 | H | $CCl_3$ | -OH | 154-156 | 4 |
| 33 | 4-F | $CHCl_2$ | $-OCH_3$ | 168 | 3a |
| 34 | 3-CN | $CCl_3$ | -OH | 155 | 4 |
| 35 | $4-Cl-C_6H_4-O-$ | $CCl_3$ | -OH | 142 | 4 |
| 36 | $2,4-Cl_2$ | $CCl_3$ | $-OC_2H_5$ | 111-112 | 8 |
| 37 | $2,4-Cl_2$ | $CCl_3$ | -OH | 156 | 4 |
| 38 | $4-Cl-C_6H_4-O-$ | $CCl_3$ | $-OCH_3$ | 1,5925 | 3b |
| 39 | $2,4-Cl_2$ | $CHCl_2$ | $-OCH_3$ | 147 | 3a |
| 40 | 2-Cl | $CHCl_2$ | -OH | 188-189 | 4 |
| 41 | 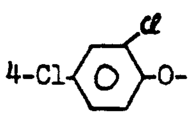 | $CCl_3$ | $-OCH_3$ | Sirup | 3b |
| 42 | 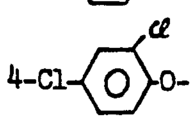 | $CCl_3$ | -OH | 141 | 4 |
| 43 | $2,4-Cl_2$ | $CCl_3$ | $-NH-C_6H_5$ | 215-217 | 6 |

Fortsetzung Tabelle I

| Bsp. | $(Z)_n$ | Y | X | Schmp. (°C). $n_D^{30}$ | Herst.gem. Beisp. Nr. |
|---|---|---|---|---|---|
| 44 | 2,4-$Cl_2$ | $CCl_3$ | $-NH-C_6H_4-3-CF_3$ | 166-167 | 6 |
| 45 | 3,5-$Cl_2$ | $CCl_3$ | " | 160-161 | 6 |
| 46 | 2,4-$Cl_2$, 5-$OCH_3$ | $CCl_3$ | " | 173 | 6 |
| 47 | 2,4-$Cl_2$ | $CCl_3$ | $-OCH_3$ | 162-163 | 3b |
| 48 | 2,4-$Cl_2$ | $CCl_3$ | $-OCH_2CH_2CH_2CH_3$ | 66-67 | 5 |
| 49 | 2-$CH_3$, 4-Cl | $CCl_3$ | 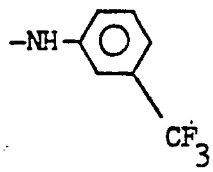 | 166 | 6 |
| 50 | 2-$CH_3$, 4-Cl | $CCl_3$ | 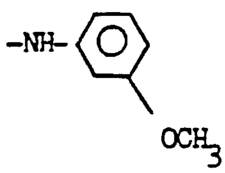 | 167-168 | 6 |
| 51 | 2,4-$Cl_2$ | $CCl_3$ | $-OCH_2-C(=O)-N(CH_3)-C_6H_5$ | 187-188 | 5* |
| 52 | 3-$CF_3$ | $CCl_3$ | $-OC_2H_5$ | 64 | 3a |
| 53 | 3,5-$Cl_2$ | $CCl_3$ | $-OCH_2-C(=O)-N(CH_3)-C_6H_5$ | 203 | 5* |
| 54 | 2-$CH_3$, 4-Cl | $CCl_3$ | 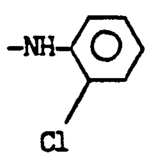 | 203 | 6 |

Fortsetzung Tabelle I

| Bsp. | $(Z)_n$ | Y | X | Schmp. (°C) $n_D^{30}$ | Herst.gem. Beisp. Nr. |
|---|---|---|---|---|---|
| 55 | 2,4-$Cl_2$ | $CCl_3$ | 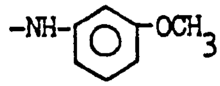 | 193-194 | 6 |
| 56 | 2,4-$Cl_2$ | $CCl_3$ | 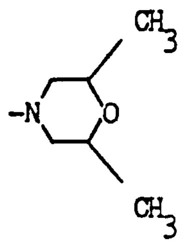 | 68-72 | 6 |
| 57 | 2,4-$Cl_2$ | $CCl_3$ | 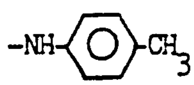 | 205-207 | 6 |
| 58 | 4-Cl | $CCl_3$ | $-OCH_3$ | 136-137 | 3b |
| 59 | 4-Cl | $CCl_3$ | -OH | 145-147 | 4 |
| 60 | 3-Cl, 4-F | $CCl_3$ | 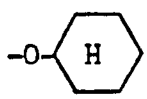 | 131-132 | 5 |
| 61 | 3-Cl, 4-F | $CCl_3$ | $-OCH_2CH(CH_3)_2$ | 122-123 | 5 |
| 62 | 2,4-$Cl_2$ | $CCl_3$ | 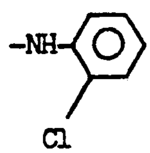 | 160-163 | 6 |
| 63 | 2,4-$Cl_2$ | $CCl_3$ | $-N(C_2H_5)_2$ | glasartig | 6 |
| 64 | 4-$CH_3$ | $CCl_3$ | -OH | 146 | 4 |
| 65 | 4-$CH_3$ | $CCl_3$ | $-OCH_3$ | 132-134 | 3b |
| 66 | 4-F | $CCl_3$ | $-OCH_3$ | 113-114 | 3b |
| 67 | 2-$CH_3$, 4-Cl | $CCl_3$ | $-OC_2H_5$ | 117-118 | 8 |
| 68 | 2-$CH_3$, 4-Cl | $CCl_3$ | $-OCH_3$ | 125-126 | 3b |
| 69 | 3-CN | $CCl_3$ | $-OCH_3$ | 125-126 | 3b |
| 70 | 2,4-$Cl_2$ | $CCl_3$ | $-OCH(CH_3)_2$ | 154-155 | 5 |
| 71 | 2,4-$Cl_2$ | $CCl_3$ | $-OCH_2CH_2CH_3$ | 129-130 | 5 |

Fortsetzung Tabelle I

| Bsp. | $(Z)_n$ | Y | X | Schmp. (°C) $n_D^{30}$ | Herst.gem. Beisp. Nr. |
|---|---|---|---|---|---|
| 72 | 4-F | $CCl_3$ | -OH | 131-132 | 4 |
| 73 | 4-F | $CCl_3$ | $-OCH_2CH_2CH_3$ | 120-121 | 5 |
| 74 | 3-Cl, 4-F | $CCl_3$ | $-OCH_3$ | 114-115 | 3b |
| 75 | $2,6-(C_2H_5)_2$ | $CCl_3$ | -OH | 193-195 | 4 |
| 76 | $2,4-Cl_2$ | $CCl_3$ | $-OCH_2CH(CH_3)_2$ | 119-121 | 5 |
| 77 | 2-Cl | $CCl_3$ | $-OCH(CH_3)_2$ | 131-132 | 5 |
| 78 | $2,4-Cl_2$, $5-OCH_3$ | $CCl_3$ | $-OCH_3$ | 155 | 3b |
| 79 | $2,4-Cl_2$, $5-OCH_3$ | $CCl_3$ | -OH | 215 | 4 |
| 80 | $2-CH_3$, 4-Cl | $CCl_3$ | -OH | 112 | 4 |
| 81 | 2-Cl | $CCl_3$ | $-OCH_2CH(CH_3)_2$ | 79-80 | 5 |
| 82 | $2-CH_3$, 4-Cl | $CCl_3$ | $-OCH_2-\underset{\overset{\|}{O}}{C}-\underset{\overset{\vert}{CH_3}}{N}-C_6H_5$ | 153 | 5* |
| 83 | 3-Cl, 4-F | $CCl_3$ | $-OC_2H_5$ | 118-119 | 8 |
| 84 | 3-Cl, 4-F | $CCl_3$ | -OH | 174 | 4 |
| 85 | 4-F | $CCl_3$ | $-OCH_2CH(CH_3)_2$ | 103-104 | 5 |
| 86 | $2-CH_3$, 4-Cl | $CCl_3$ | $-OCH_2CH_2CH_2CH_3$ | 112 | 5 |
| 87 | 4-Cl | $CCl_3$ | $-OC_2H_5$ | 161-162 | 8 |
| 88 | $4-CH_3$ | $CCl_3$ | $-OCH(CH_3)_2$ | 154-155 | 5 |
| 89 | H | $CHCl_2$ | -OH | 185 | 4 |
| 90 | $2,4-Cl_2$ | $CF_3$ | $-OCH_3$ | 131 | 3b |
| 91 | $4-CH_3$ | $CCl_3$ | $-OC_2H_5$ | 104-105 | 8 |
| 92 | $4-CH_3$ | $CCl_3$ | $-OCH_2CH(CH_3)_2$ | 125-126 | 5 |
| 93 | 4-Cl- 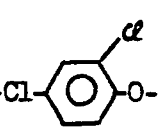 -O- | $CCl_3$ | $-OC_2H_5$ | 147 | 8 |
| 94 | " | $CCl_3$ | $-OCH_2CH_2CH_3$ | 99 | 5 |

Fortsetzung Tabelle I

| Bsp. | $(Z)_n$ | Y | X | Schmp. (°C) $n_D^{30}$ | Herst.gem. Beisp. Nr. |
|---|---|---|---|---|---|
| 95 | 4-F | $CF_3$ | $-OCH_3$ | 87-88 | 3b |
| 96 | 4-Cl | $CCl_3$ | $-OCH_2CH(CH_3)_2$ | 138-139 | 5 |
| 97 | 2,6-$(C_2H_5)_2$ | $CCl_3$ | $-OC_2H_5$ | 96-97 | 8 |
| 98 | 3,4-$Cl_2$ | $CCl_3$ | $-OCH_3$ | 133 | 3b |
| 99 | 2,6 $(C_2H_5)_2$ | $CCl_3$ | -ONa | 233-234 | 4 |
| 100 | 2,6 $(C_2H_5)_2$ | $CCl_3$ | $-OCH_2CH_2CH_3$ | 1,5390 | 5 |
| 101 | 2,6 $(C_2H_5)_2$ | $CCl_3$ | $-OCH(CH_3)_2$ | 83-84 | 5 |
| 102 | 2,6 $(C_2H_5)_2$ | $CCl_3$ | $-OCH_2CH(CH_3)_2$ | 1,5147 | 5 |
| 103 | 3,4-$Cl_2$ | $CCl_3$ | OH | 167-168 | 4 |
| 104 | 2-$CF_3$,4-Cl | $CCl_3$ | $OCH_3$ | 110 | 3a |
| 105 | 2-$CF_3$,4-Cl | $CCl_3$ | OH | 143,145 | 4 |
| 106 | 2-F,4-Cl-5-$OCH_3$ | $CCl_3$ | $OCH_3$ | 177-178 | 3a |
| 107 | 2-F,4-Cl,5-$OCH_3$ | $CCl_3$ | OH | 176 | 4 |
| 108 | 2-$CHF_2CF_2O$- | $CCl_3$ | $OCH_3$ | 116-117 | 3a |
| 109 | 3-CHClF-$CF_2O$- | $CCl_3$ | $OCH_3$ | Sirup | 3a |
| 110 | 2-$CH_3$,3-Cl | $CCl_3$ | $OCH_3$ | 144-145 | 3a |
| 111 | 2,6-$Cl_2$ | $CCl_3$ | $OCH_3$ | 143-144 | 3a |
| 112 | 2,5-$(OCH_3)_2$,4-Cl | $CCl_3$ | $OCH_3$ | 149 | 3a |
| 113 | 2,5-$(OCH_3)_2$,4-Cl | $CCl_3$ | OH | 223-224 | 4 |
| 114 | 3,5-$Cl_2$,4-$OCH_3$ | $CCl_3$ | $OCH_3$ | 148-149 | 3a |
| 115 | 3,5-$Cl_2$-4-$OCH_3$ | $CCl_3$ | OH | 150 | 4 |
| 116 | 2-Cl,4-Br | $CCl_3$ | $OC_2H_5$ | 113-114 | 3a |
| 117 | 2-Cl,4-Br | $CCl_3$ | OH | 220 | 4 |
| 118 | 2-Br | $CCl_3$ | $OCH_3$ | 126-127 | 3a |
| 119 | 2-Br | $CCl_3$ | OH | 195-196 | 4 |
| 120 | 2,4-$Cl_2$ | $CCl_3$ | $OCH_2CH_2Cl$ | 88-89 | 5 |
| 121 | 3-CHClF-$CF_2O$- | $CCl_3$ | OH | 128-130 | 4 |
| 122 | 2-$CH_3$,4-Cl | $CCl_3$ | $CH_3$ | 141 | 14 |
| 123 | 3-$CH_3$,4-$CHF_2CF_2O$ | $CCl_3$ | $CH_3$ | 119-120 | 14 |

Fortsetzung Tabelle I

| Bsp. | $(Z)_n$ | Y | X | Schmp. (°C) $n_D^{30}$ | Herst.gem. Beisp. Nr. |
|---|---|---|---|---|---|
| 124 | 2,4-$Cl_2$ | $CCl_3$ | $CH_3$ | 120-121 | 14 |
| 125 | 3-$OCF_3$ | $CCl_3$ | $CH_3$ | 92-93 | 14 |
| 126 | 3-$CHCl_2CF_2O$ | $CCl_3$ | $CH_3$ | 114-115 | 14 |
| 127 | H | $CCl_3$ | $CH_3$ | 139-140 | 14 |
| 128 | 2-Cl | $CCl_3$ | $CH_3$ | 121-122 | 14 |
| 129 | 3-$CF_2Cl$ | $CCl_3$ | $CH_3$ | Sirup | 14 |
| 130 | 4-Cl | $(CH_3)_2C=CH-$ | $OCH_3$ | 118-119 | 9 |
| 131 | 2-$CH_3$,4-Cl | $(CH_3)_2C=CH-$ | $OCH_3$ | 142-143 | 9 |
| 132 | 2-Cl | $CH_2Cl$ | $OCH_3$ | 112-113 | 10 |
| 133 | 2-$CH_3$,4-Cl | $CH_3$ | $OCH_3$ | 73 | 10 |
| 134 | 3-Cl,4-F | $(CH_3)_2C=CH-$ | $OCH_3$ | 185-186 | 9 |
| 135 | 2,4-$Cl_2$,5-$OCH_3$ | $(CH_3)_2C=CH-$ | $OCH_3$ | 200-201 | 9 |
| 136 | 3,4-$Cl_2$ | $CHCl_2-CF_2-$ | $OC_2H_5$ | $n_D^{30}$ :1,5458 | 9 |
| 137 | 2-$CH_3$,4-Cl | $CHCl_2-CF_2-$ | $OC_2H_5$ | $n_D^{30}$ :1,5272 | 9 |
| 138 | 2-$CH_3$,4-Cl | $CHF_2-CF_2-$ | $OC_2H_5$ | $n_D^{30}$ :1,4991 | 9 |
| 139 | 4-F | $CHF_2-CF_2-$ | $OCH_3$ | 66-67 | 9 |
| 140 | 2-Cl | $(CH_3)_2C=CH-$ | OH | 194 | 4 |
| 141 | 3,4-$Cl_2$ | $CF_2H-CF_2-$ | $OC_2H_5$ | $n_D^{30}$ :1,5198 | 9 |
| 142 | 2,4-$Cl_2$,5-$OCH_3$ | $CF_2H-CF_2-$ | $OCH_3$ | 112-113 | 9 |
| 143 | 2-$CH_3$,4-Cl | $CH_3CH=CH-$ | $OCH_3$ | 175-176 | 9 |
| 144 | 4-F | 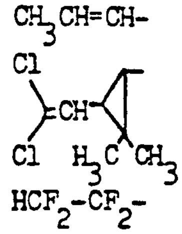 | $OCH_3$ | 149-160 | 9 |
| 145 | 3-$CF_3$ | $HCF_2-CF_2-$ | $OCH_3$ | Sirup | 9 |
| 146 | 2,4-$Cl_2$ | $HCF_2-CF_2-$ | $OC_2H_5$ | Sirup | 9 |

Fortsetzung Tabelle I

| Bsp. | $(Z)_n$ | Y | X | Schmp. (°C) $n_D^{30}$ | Herst.gem. Beisp. Nr. |
|---|---|---|---|---|---|
| 147 | 2,4-$Cl_2$ | $HCF_2-(CF_2)_3-$ | $OC_2H_5$ | 115-116 | 9 |
| 148 | 2,4-$Cl_2$ | $BrCF_2-CF_2-$ | $OC_2H_5$ | 100-101 | 9 |
| 149 | 2,4-$Cl_2$ | $HCF_2-CF_2-$ | OH | 123-124 | 9 |
| 150 | 2-Cl | $CH_3$ | OH | 190 | 11b |
| 151 | 2-Cl | $CH_3$ | $OC_2H_5$ | 110-111 | 11c |
| 152 | 4-Cl | $CH_3$ | OH | 172 | 11b |
| 153 | 4-Cl | $CH_3$ | $OC_2H_5$ | 115 | 11c |
| 154 | 3-Cl,4-F | $CH_3$ | OH | 183 | 11b |
| 155 | 4-F | $CH_3$ | OH | 177 | 11b |
| 156 | 2-$CH_3$,4-Cl | $CH_3$ | OH | 177-178 | 11b |
| 157 | 2,4-$Cl_2$,5-$OCH_3$ | $CH_3$ | OH | 193-194 | 11b |
| 158 | 2,4-$Cl_2$ | $CH_3$ | $OCH_3$ | 180-181 | 11c |
| 159 | 3-Cl,4-F | $CH_3$ | $OCH_3$ | 140-142 | 11c |
| 160 | 3-Cl,4-F | $CH_3$ | $OC_2H_5$ | 114-115 | 11c |
| 161 | 2,6-$(C_2H_5)_2$ | $CHF_2-CF_2-$ | OH | 222-223 | 4 |
| 162 | 4-F | $CH_3$ | $OC_2H_5$ | 105-106 | 11c |
| 163 | 2-$CH_3$,4-Cl | $CH_3$ | $OC_2H_5$ | 153-154 | 11c |
| 164 | 2,4-$Cl_2$ | H | OH | 185-186 | 4 |
| 165 | 2,4-$Cl_2$ | H | $OC_2H_5$ | 105-106 | 13 |
| 166 | 4-$CH_3$ | $CH_3$ | $OC_2H_5$ | 1,5466 | 11c |
| 167 | 4-$CH_3$ | $CH_3$ | OH | 183 | 11b |
| 168 | 2-Cl,4-Br | $CH_3$ | $OC_2H_5$ | 142-143 | 11c |
| 169 | 2-Cl,4-Br | $CH_3$ | OH | 172-173 | 11b |
| 170 | 3-$CF_3$ | $CH_3$ | OH | 164-165 | 11b |
| 171 | 2,4-$Cl_2$ | $CH_3$ | $OC_2H_5$ | 133-134 | 11c |
| 172 | 2,4-$Cl_2$ | $CH_3$ | OH | 163-164 | 11b |

*) in Toluol mit äquimolarer Menge des Alkohols und Triethylamin als Säurebinder.

C. Biologische Beispiele

Beispiel 1

Weizen wurde im Gewächshaus in Töpfen von 9 cm ø bis zum 3—4 Blattstadium herangezogen und dann mit dem Herbizid und den erfindungsgemäßen Verbindungen behandelt. Herbizide und die Verbindungen der Formel I wurden dabei in Form wässriger Suspensionen bzw. Emulsionen mit einer Wasseraufwandmenge von umgerechnet 800 l/ha ausgebracht. 3 Wochen nach der Behandlung wurden die Pflanzen auf jede Art von Schädigung durch die ausgebrachten Herbizide bonitiert, wobei insbesondere das Ausmaß der anhaltenden Wachstumshemmung berücksichtigt wurde.

Die Ergebnisse aus Tabelle 1 veranschaulichen, daß die erfindungsgemäßen Verbindungen starke Herbizidschäden sehr effektiv reduzieren können.

Selbst bei starken Überdosierungen des Herbizids H von 2,0 kg AS/ha werden auftretende herbizide Schadsymtome stark reduziert, so daß nur noch geringe Dauerschäden zurückbleiben. Damit wird die gute Safener-Wirkung verdeutlicht. Naturgemäß werden geringe Schäden völlig aufgehoben (siehe Beispiel 2). Mischungen aus Herbiziden und erfindungsgemäßen Verbindungen eignen sich also zur selektiven Umkrautbekämpfung in Getreide.

Tabelle 1

| Verbindungen H+ | Beisp.Nr. | Dosis kg a.i./ha | Herbizide Wirkung in % TA |
|---|---|---|---|
| H | - | 2,0 | 75 |
| H + | 17 | 2,0 + 2,5 | 20 |
| H + | 22 | " | 30 |
| H + | 7 | " | 30 |
| H + | 27 | " | 25 |
| H + | 15 | " | 11 |
| H + | 16 | " | 24 |
| H + | 36 | " | 13 |
| H + | 67 | " | 10 |
| H + | 25 | " | 25 |
| H + | 78 | " | 18 |
| H + | 31 | " | 20 |
| H + | 69 | " | 28 |
| H + | 3 | " | 26 |
| H + | 79 | " | 30 |
| H + | 80 | " | 30 |
| H + | 32 | " | 24 |
| H + | 37 | " | 24 |
| H + | 19 | " | 25 |
| H + | 43 | " | 30 |
| H + | 63 | " | 20 |
| H + | 18 | " | 13 |
| H + | 29 | " | 20 |
| H + | 33 | " | 20 |
| H + | 39 | " | 20 |
| H + | 41 | " | 20 |
| H + | 64 | " | 20 |
| H + | 66 | " | 15 |
| H + | 73 | " | 25 |
| H + | 85 | " | 20 |
| H + | 74 | " | 25 |
| H + | 127 | 2,0 + 1,0 | 30 |

Tabelle 1

| Verbindungen H+ | Beisp.Nr. | Dosis kg a.i./ha | Herbizide Wirkung in % TA |
|---|---|---|---|
| H + | 89 | 2,0 + 2,5 | 30 |
| H + | 90 | " | 50 |
| H + | 146 | " | 30 |
| H + | 9 | " | 40 |
| H + | 160 | " | 60 |
| H + | 163 | " | 30 |
| H + | 20 | " | 13 |
| H + | 21 | " | 22 |
| H + | 47 | " | 22 |
| H + | 48 | " | 7 |
| H + | 70 | " | 10 |
| H + | 71 | " | 50 |
| H + | 76 | " | 20 |
| H + | 68 | " | 10 |
| H + | 72 | " | 22 |
| H + | 73 | " | 40 |
| H + | 84 | " | 35 |
| H + | 8 | " | 30 |
| H + | 75 | " | 50 |
| H + | 59 | " | 20 |
| H + | 83 | " | 28 |
| H + | 52 | " | 60 |
| H + | 5 | " | 22 |
| H + | 77 | " | 20 |
| H + | 81 | " | 30 |
| H + | 92 | " | 40 |
| H + | 91 | " | 40 |
| H + | 88 | " | 50 |
| H + | 86 | " | 40 |
| H + | 87 | " | 20 |
| H + | 96 | " | 40 |
| H + | 100 | " | 40 |
| H + | 103 | " | 30 |
| H + | 102 | " | 40 |

Tabelle 1

| Verbindungen H+ | Beisp.Nr. | Dosis kg a.i./ha | Herbizide Wirkung in % TA |
|---|---|---|---|
| H + | 104 | 2,0 + 2,5 | 50 |
| H + | 106 | " | 50 |
| H + | 107 | " | 50 |
| H + | 110 | " | 48 |
| H + | 111 | " | 50 |
| H + | 116 | " | 20 |
| H + | 118 | " | 30 |
| H + | 113 | " | 60 |
| H + | 115 | " | 40 |
| H + | 117 | " | 18 |
| H + | 119 | " | 50 |
| H + | 120 | " | 20 |
| H + | 137 | " | 25 |
| H + | 138 | " | 25 |
| H + | 139 | " | 25 |
| H + | 141 | " | 40 |
| H + | 142 | " | 30 |
| H + | 143 | " | 40 |
| H + | 133 | " | 50 |
| H + | 134 | " | 50 |
| H + | 135 | " | 50 |
| H + | 148 | " | 20 |
| H + | 149 | " | 55 |
| H + | 11c | " | 40 |
| H + | 11b | " | 25 |
| H + | 150 | " | 35 |
| H + | 151 | " | 30 |
| H + | 152 | " | 50 |
| H + | 153 | " | 27 |
| H + | 154 | " | 27 |
| H + | 155 | " | 50 |
| H + | 156 | " | 25 |
| H + | 157 | " | 43 |
| H + | 158 | " | 20 |
| H + | 171 | " | 20 |

Abkürzungen Tabelle 1
TA = Triticum aestivum
a.1. = Aktivsubstanz
H = Fenoxaprop-ethyl = 2-(4-(6-Chlorbenzoxazol-2-yloxy)phenoxy)propionsäureethylester

Beispiel 2

Weizen und die beiden Schadgräser Alopecurus myoruoides und Avena fatua wurden in Töpfen von 9 cm ø im lehmigem Sandboden ausgesät, unter kühlen Bedingungen bis zum Anfang der Bestockung im Gewächshaus angezogen und mit den erfindungsgemäßen Verbindungen behandelt. Die Präparate wurden in Form wässriger Emulsionen bzw. Suspensionen mit einer Wasseraufwandmenge von 300 l/ha gemeinsam mit dem Herbizid (tank mix) ausgebracht.

4 Wochen nach Applikation wurden die Versuchspflanzen auf Wachstumsveränderungen und Schädigung bonitiert.

Die Ergebnisse aus Tabelle 2 zeigen, daß die erfindungsgemäßen Verbindungen sehr gute Safenereigenschaft haben und Herbizid-Schäden, die an Kulturpflanzen wie z.B. Getreide entstehen, wirkungsvoll verhindern können, ohne die eigentliche herbizide Wirkung gegen Schadgräser zu beeinträchtigen.

Mischungen aus Herbiziden und erfindungsgemäßen Verbindungen können somit zur selektiven Umkrautbekämpfung eingesetzt werden.

Tabelle 2

| Herbizid H + | Verb. aus Beispiel-Nr. | Dosis kg AS/ha H + Vbg. aus Bsp.Nr. | % Schädigung an Weizen (TA) | % Herb. Wirkung gegen ALM | AVF |
|---|---|---|---|---|---|
| Herbizid H | - | 0,8 | 52 | - | - |
| | | 0,4 | 40 | 100 | 100 |
| | | 0,2 | 18 | 98 | 100 |
| H + | 7 | 0,8 + 0,4 | 2 | - | - |
| | | 0,4 + 0,2 | 0 | 100 | 100 |
| | | 0,2 + 0,1 | 0 | 98 | 100 |
| H + | 27 | " | 2 | - | - |
| | | | 0 | 100 | 100 |
| | | | 0 | 99 | 99 |
| H + | 15 | " | 2 | - | - |
| | | | 0 | 100 | 100 |
| | | | 0 | 100 | - |
| H + | 16 | " | 1 | - | - |
| | | | 0 | 100 | 100 |
| | | | 0 | 98 | 98 |
| H + | 18 | " | 3 | - | - |
| | | | 0 | 100 | 100 |
| | | | 0 | 100 | 97 |
| H + | 37 | " | 2 | - | - |
| | | | 0 | 100 | 100 |
| | | | 0 | 99 | 100 |
| H + | 36 | " | 2 | - | - |
| | | | 0 | 100 | 100 |
| | | | 0 | 97 | 100 |

Tabelle 2

| Herbizid H + | Verb. aus Beispiel-Nr. | Dosis kg AS/ha H + Vbg. aus Bsp.Nr. | % Schädigung an Weizen (TA) | % Herb. Wirkung gegen ALM | AVF |
|---|---|---|---|---|---|
| H + | 78 | 0,8 + 0,4 | 2 | - | - |
| | | 0,4 + 0,2 | 0 | 100 | 100 |
| | | 0,2 + 0,1 | 0 | 100 | 100 |
| H + | 67 | " | 2 | - | - |
| | | | 0 | 100 | 100 |
| | | | 0 | 100 | 100 |
| H + | 22 | " | 2 | - | - |
| | | | 0 | 100 | 100 |
| | | | 0 | 100 | 99 |
| H + | 79 | " | 3 | - | - |
| | | | 2 | 100 | 100 |
| | | | 0 | 98 | 98 |
| H + | 80 | " | 4 | - | - |
| | | | 2 | 100 | 100 |
| | | | 0 | 98 | 100 |
| | | | 0 | - | - |
| H + | 63 | | 0 | 100 | 100 |
| | | | 0 | 99 | 97 |
| H + | 19 | " | 2 | - | - |
| | | | 0 | 100 | 100 |
| | | | 0 | 100 | 97 |

ALM = Alopecurus myouroides
AVF = Avena fatua
H = siehe Tabelle 1

Beispiel 3

Gerste (Sorte Oriol) wurde in Töpfen von 13 cm ø in lehmigem Sandboden ausgesät und unter Freilandklimabedingungen bis zum Anfang der Bestockung angezogen und mit den Mischungen (Tankmix) aus dem Herbizid und den erfindungsgemäßen Verbindungen behandelt. Die Präparate wurden in Form wäßriger Emulsionen bzw. Suspensionen mit einer Wasseraufwandmenge von 300 l/ha ausgebracht.

2 Wochen nach Applikation wurden die Versuchspflanzen auf Wachstumsveränderungen und andere Schädigungen bonitiert.

Wie die Ergebnisse aus Tabelle 3 zeigen, haben die erfindungsgemäßen Verbindungen sehr gute Safenereigenschaften und können somit Herbizidschäden, die an Kulturpflanzen, wie z.B. Gerste, entstehen, wirkungsvoll verhindern, ohne den eigentlichen Bekämpfungserfolg gegen Schadgräser zu beeinträchtigen.

Tabelle 3

| Verbindung $H_1$ + Bsp. Nr. | Dosis (kg a.i./ha) | herbizide Wirkung in % HV |
|---|---|---|
| $H_1$ | 3,0 | 30 |
| | 1,5 | 13 |
| $H_1$ + 36 | 3,0 + 0,3 | 15 |
| | 1,5 + 0,15 | 3 |

Abkürzungen: HV= Hordeum vulgare

$H_1$= Diclofop-methyl [2-(4-(2,4-Dichlorphenoxy)-phenoxy)propionsäuremethylester]

**Patentansprüche für die Vertragsstaaten: BE CH DE FR GB IT LI NL**

1. Pflanzenschützende Mittel, dadurch gekennzeichnet, daß sie eine Verbindung der Formel I,

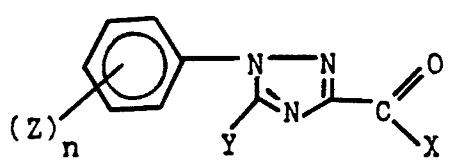

(I),

worin

Z bei gleicher oder verschiedener Bedeutung Halogen, Nitro, Cyano, Trifluormethyl, $(C_1-C_4)$-Alkyl, $(C_1-C_4)$Alkoxy, $(C_1-C_4)$Alkylthio, wobei die Alkyl-, Alkoxy- und Alkylthiogruppen durch ein oder mehrere Halogenatome, insbesondere Fluor oder Chlor, substituiert sein können, $(C_3-C_6)$Cycloalkyl, das durch $(C_1-C_4)$Alkyl substituiert sein kann, Phenyl oder Phenoxy, wobei Phenyl und Phenoxy durch Halogen ein- oder mehrfach und/oder einfach durch Trifluormethyl substituiert sein können,

Y Wasserstoff, $(C_1-C_4)$Alkyl, das ganz oder teilweise durch Halogenatome und/oder einfach durch $(C_1-C_4)$Alkoxy oder $(C_1-C_4)$Alkylthio substituiert sein kann, $(C_2-C_6)$Alkenyl, $(C_2-C_6)$Alkinyl, oder $(C_3-C_6)$Cycloalkyl, das durch $(C_1-C_4)$Alkyl und/oder einen Dichlorvinylrest substituiert sein kann,

X Hydroxy, $(C_1-C_4)$Alkyl, $(C_3-C_6)$Cycloalkoxy, Phenyl$(C_1-C_6)$alkoxy, Phenoxy, $(C_2-C_6)$Alkenyloxy, $(C_2-C_6)$Alkinyloxy, $(C_1-C_6)$Alkoxy, $(C_1-C_6)$Alkylthio, wobei die Alkoxy oder Alkylthiogruppe durch $(C_1-C_2)$Alkoxy, Mono- oder Di-$(C_1-C_4)$Alkylaminocarbonyl, Phenylaminocarbonyl, N-$(C_1-C_4)$Alkyl-phenylaminocarbonyl, Mono- oder Di-$(C_1-C_6)$Alkylamino, $(C_1-C_6)$Alkyl-carbonyloxy, $(C_1-C_2)$Alkylthio, Cyano oder Halogen substituiert sein kann, einen Rest der Formeln

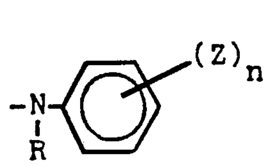 oder 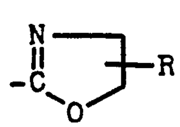 ,

worin jeweils R Wasserstoff oder $(C_1-C_4)$Alkyl bedeutet, Mono oder Di-$(C_1-C_4)$Alkylamino, $(C_5-C_6)$Cycloalkylamino, Piperidino, Morpholino oder 2,6-Dimethylmorpholino bedeuten, einen Rest der Formel

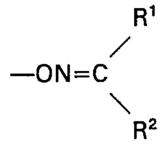,

worin $R^1$ und $R^2$ gleich oder verschieden sein können und $(C_1—C_4)$Alkylreste bedeuten, worin $R^1$ und $R^2$ auch gemeinsam einen 5-, 6- oder 7-gliedrigen Cycloalkylrest bilden können, und

n die Zahl 0, 1, 2 oder 3 bedeuten oder im Falle X = OH deren für die Landwirtschaft einsetzbare Salze enthalten.

2. Mittel gemäß Anspruch 1, dadurch gekennzeichnet, daß sie eine Verbindung der Formel I von Anspruch 1, worin

Y = $(C_1—C_2)$Alkyl, das ganz oder teilweise durch F, Cl oder Br substituiert sein kann,

Z = bei gleicher oder verschiedener Bedeutung Halogen, $(C_1—C_4)$Alkoxy, $(C_1—C_4)$Alkyl oder $CF_3$,

X = $(C_1—C_6)$Alkoxy oder Hydroxy und n = 1, 2 oder 3 bedeuten, enthalten.

3. Mittel gemäß Anspruch 2, dadurch gekennzeichnet, daß Y = $CCl_3$, $CHCl_2$, $CHF_2CF_2$ oder $CH_3$ bedeutet.

4. Mittel gemäß Ansprüchen 1, 2 oder 3, dadurch gekennzeichnet, daß sie in Kombination mit einer Verbindung der Formel I von Ansprüchen 1, 2 oder 3 zusätzlich ein Herbizid aus der Gruppe der Thiolcarbamate, Carbamate, Halogenacetanilide, Phenoxy-, Naphthoxy- oder Phenoxyphenoxycarbonsäureester, der Dimedonoximderivate oder der Heteroaryloxyphenoxycarbonsäureester, letztere ausgewählt aus Chinolyloxy-, Chinoxalyloxy-, Pyridyloxy-, Benzoxazolyloxy- oder Benzthiazolyloxyphenoxy-carbonsäureester, enthalten.

5. Verfahren zum Schutz von Kulturpflanzen gegen phytotoxische Nebenwirkungen von Herbiziden, dadurch gekennzeichnet, daß man die Pflanzen, Pflanzensamen oder Anbauflächen mit einer wirksamen Menge einer Verbindung der Formel I von Ansprüchen 1, 2 oder 3 vor, nach oder gleichzeitig mit dem Herbizid behandelt.

6. Verfahren gemäß Anspruch 5, dadurch gekennzeichnet, daß man als Herbizid ein Thiolcarbamat, Carbamat, Halogenacetanilid, Phenoxy-, Naphthoxy- oder Phenoxyphenoxycarbonsäureester, ein Dimedonoximderivat oder einen Heteroaryloxyphenoxy-carbonsäureester, letzter ausgewählt aus Chinolyloxy-, Chinoxalyloxy-, Pyridiyloxy-, Benzoxazolyloxy- oder Benzthiazolyloxyphenoxy-carbonsäureester, einsetzt.

7. Verwendung von Verbindungen der Formel I von Ansprüchen 1 oder 2 zum Schutz von Kulturpflanzen gegen phytotoxische Nebenwirkungen von Herbiziden.

8. Verbindungen der nach Anspruch 1, 2 oder 3 definierten Formel I, ausgenommen solche, in welchen

a) y = H, $(Z)_n$ = H und X = OH, $OCH_3$, $OC_2H_5$ oder $NHC_6H_5$,
b) y = H, $(Z)_n$ = 4-$OCH_3$ und X = OH, $OCH_3$ oder $NHC_6H_5$,
c) y = H, $(Z)_n$ = 3-$OCH_3$, 4-Cl, 4-$CH_3$ und X = $OCH_3$,
d) y = H, $(Z)_n$ = 3-Cl und X = OH,
e) y = $CH_3$, $(Z)_n$ = H oder $OCH_3$ und X = OH,
f) y = $CH_3$, $(Z)_n$ = 4-$NO_2$ und X = OH, $OCH_3$ oder $OC_2H_5$,
g) y = $CH_3$, $(Z)_n$ = 2-Cl, 4-$NO_2$—2—$CH_3$ oder 4-$NO_2$—2—$OCH_3$ und X = $OC_2H_5$,
h) y = $C_2H_5$, $(Z)_n$ = H und X = OH
i) y = n-$C_3H_7$ oder i-$C_3H_7$, $(Z)_n$ = H und X = OH, $OCH_3$ oder $OC_2H_5$
k) x = $CH_3$, y = H oder $C_2H_5$ und $(Z)_n$ = H, 4-Cl oder 4-$OC_2H_5$ oder
l) x = $CH_3$, y = $CH_3$ und $(Z)_n$ = H, 4-Cl, 4-$OCH_3$, 4-$OC_2H_5$ oder 4-$NO_2$

bedeuten.

9. Verfahren zur Herstellung der Verbindung von Anspruch 8, dadurch gekennzeichnet, daß man

a) eine Verbindung der Formel II

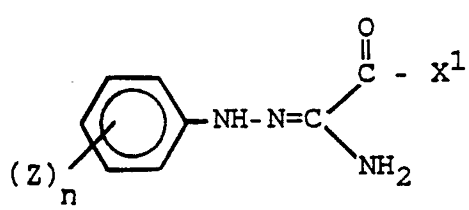 (II),

worin $X^1$ die Bedeutung von X außer Hydroxy besitzt,

$a_1$) mit einer Verbindung der Formel Y—CO—Cl oder
$a_2$) mit einem Säureanhydrid der Formel Y—CO—O—CO—Y oder
$a_3$) mit einem Orthoester der Formel Y—C$(OR^1)_3$, worin $R^1$ = $(C_1—C_4)$Alkyl bedeutet, oder
b) für Verbindungen mit Y = H, $(C_1—C_4)$Alkyl eine Verbindung der Formel III

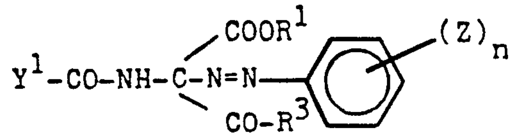 (III),

worin $Y^1$ = H oder $(C_1—C_4)$Alkyl und $R^3$ = $(C_1—C_4)$Alkoxy oder $CH_3$ bedeuten, mit einer Base umsetzt, wobei die unter $a_1$) und $a_2$) erhaltenen Verbindungen gegebenenfalls in Essigsäure erhitzt werden, und die erhaltenen Verbindungen der Formel I gegebenenfalls durch Derivatisierung in andere Verbindungen der Formel I oder deren Salze überführt werden.

10. Verbindungen der Formel I gemäß Anspruch 8, worin Y = $CCl_3$, $CHCl_2$; X = $(C_1—C_4)$-Alkoxy, OH; Z = Cl, F, $OCH_3$ und n die Zahl 1, 2 oder 3 bedeuten.

11. Verbindungen der Formel I gemäß Anspruch 8, worin Y = $CCl_3$; X = $(C_1—C_4)$-Alkoxy und $(Z)_n$ = 2,4-$Cl_2$ bedeuten.

12. Verbindung der Formel I gemäß Anspruch 8, worin Y = $CCl_3$; X = $OC_2H_5$ und $(Z)_n$ = 2,4-$Cl_2$ bedeuten.

**Patentansprüche für den Vertragsstaat: AT**

1. Pflanzenschützende Mittel, dadurch gekennzeichnet, daß sie eine Verbindung der Formel I,

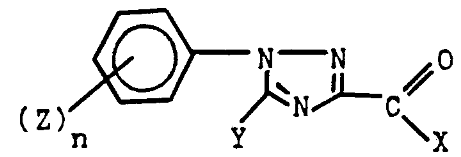 (I),

worin

Z bei gleicher oder verschiedener Bedeutung Halogen, Nitro, Cyano, Trifluormethyl, $(C_1—C_4)$-Alkyl, $(C_1—C_4)$Alkoxy, $(C_1—C_4)$Alkylthio, wobei die Alkyl-, Alkoxy- und Alkylthiogruppen durch ein oder mehrere Halogenatome, insbesondere Fluor oder Chlor, substituiert sein können, $(C_3—C_6)$Cycloalkyl, das durch $(C_1—C_4)$Alkyl substituiert sein kann, Phenyl oder Phenoxy, wobei Phenyl und Phenoxy durch Halogen ein- oder mehrfach und/oder einfach durch Trifluormethyl substituiert sein können,

Y Wasserstoff, $(C_1—C_4)$Alkyl, das ganz oder teilweise durch Halogenatome und/oder einfach durch $(C_1—C_4)$Alkoxy oder $(C_1—C_4)$Alkylthio substituiert sein kann, $(C_2—C_6)$Alkenyl, $(C_2—C_6)$Alkinyl, oder $(C_3—C_6)$Cycloalkyl, das durch $(C_1—C_4)$Alkyl und/oder einen Dichlorvinylrest substituiert sein kann,

X Hydroxy, $(C_1—C_4)$Alkyl, $(C_3—C_6)$Cycloalkoxy, Phenyl$(C_1—C_6)$alkoxy, Phenoxy, $(C_2—C_6)$Alkenyloxy, $(C_2—C_6)$Alkinyloxy, $(C_1—C_6)$Alkoxy, $(C_1—C_6)$Alkylthio, wobei die Alkoxy oder Alkylthiogruppe durch $(C_1—C_2)$Alkoxy, Mono- oder Di-$(C_1—C_4)$Alkylaminocarbonyl, Phenylaminocarbonyl, N-$(C_1—C_4)$Alkyl-phenylaminocarbonyl, Mono- oder Di-$(C_1—C_6)$Alkylamino, $(C_1—C_6)$Alkyl-carbonyloxy, $(C_1—C_2)$Alkylthio, Cyano oder Halogen substituiert sein kann, einen Rest der Formeln

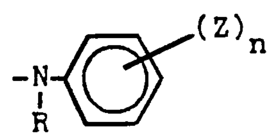 oder 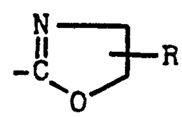 ,

worin jeweils R Wasserstoff oder $(C_1—C_4)$Alkyl bedeutet, Mono oder Di-$(C_1—C_4)$Alkylamino, $(C_5—C_6)$Cycloalkylamino, Piperidino, Morpholino oder 2,6-Dimethylmorpholino bedeuten, einen Rest der Formel

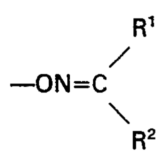 ,

worin $R^1$ und $R^2$ gleich oder verschieden sein können und $(C_1—C_4)$Alkylreste bedeuten, worin $R^1$ und $R^2$ auch gemeinsam einen 5-, 6- oder 7-gliedrigen Cycloalkylrest bilden können, und

n die Zahl 0, 1, 2 oder 3 bedeuten oder im Falle X = OH deren für die Landwirtschaft einsetzbare Salze enthalten.

2. Mittel gemäß Anspruch 1, dadurch gekennzeichnet, daß sie eine Verbindung der Formel I von Anspruch 1, worin

Y = $(C_1—C_2)$Alkyl, das ganz oder teilweise durch F, Cl oder Br substituiert sein kann,

Z = bei gleicher oder verschiedener Bedeutung Halogen, $(C_1—C_4)$Alkoxy, $(C_1—C_4)$Alkyl oder $CF_3$,

X = $(C_1—C_6)$Alkoxy oder Hydroxy und n = 1, 2 oder 3 bedeuten, enthalten.

3. Mittel gemäß Anspruch 2, dadurch gekennzeichnet, daß Y = $CCl_3$, $CHCl_2$, $CHF_2CF_2$ oder $CH_3$ bedeutet.

4. Mittel gemäß Ansprüchen 1, 2 oder 3, dadurch gekennzeichnet, daß sie in Kombination mit einer Verbindung der Formel I von Ansprüchen 1, 2 oder 3 zusätzlich ein Herbizid aus der Gruppe der Thiolcarbamate, Carbamate, Halogenacetanilide, Phenoxy-, Naphthoxy- oder Phenoxyphenoxycarbonsäureester, der Dimedonoximderivate oder der Heteroaryloxyphenoxycarbonsäureester, letztere ausgewählt aus Chinolyloxy-, Chinoxalyloxy-, Pyridyloxy-, Benzoxazolyloxy- oder Benzthiazolyloxyphenoxy-carbonsäureester, enthalten.

5. Verfahren zum Schutz von Kulturpflanzen gegen phytotoxische Nebenwirkungen von Herbiziden, dadurch gekennzeichnet, daß man die Pflanzen, Pflanzensamen oder Anbauflächen mit einer wirksamen Menge einer Verbindung der Formel I von Ansprüchen 1, 2 oder 3 vor, nach oder gleichzeitig mit dem Herbizid behandelt.

6. Verfahren gemäß Anspruch 5, dadurch gekennzeichnet, daß man als Herbizid ein Thiolcarbamat, Carbamat, Halogenacetanilid, Phenoxy-, Naphthoxy- oder Phenoxyphenoxycarbonsäureester, ein Dimedonoximderivat oder einen Heteroaryloxyphenoxy-carbonsäureester, letzter ausgewählt aus Chinolyloxy-, Chinoxalyloxy-, Pyridiyloxy-, Benzoxazolyloxy- oder Benzthiazolyloxyphenoxy-carbonsäureester, einsetzt.

7. Verwendung von Verbindungen der Formel I von Ansprüchen 1 oder 2 zum Schutz von Kulturpflanzen gegen phytotoxische Nebenwirkungen von Herbiziden.

8. Verfahren zur Herstellung von Verbindungen der nach Anspruch 1, 2 oder 3 definierten Formel I, wobei solche in denen

a) y = H, $(Z)_n$ = H und X = OH, $OCH_3$, $OC_2H_5$ oder $NHC_6H_5$,
b) y = H, $(Z)_n$ = 4-$OCH_3$ und X = OH, $OCH_3$ oder $NHC_6H_5$,
c) y = H, $(Z)_n$ = 3-$OCH_3$, 4-Cl, 4-$CH_3$ und X = $OCH_3$,
d) y = H, $(Z)_n$ = 3-Cl und X = OH,
e) y = $CH_3$, $(Z)_n$ = H oder $OCH_3$ und X = OH,
f) y = $CH_3$, $(Z)_n$ = 4-$NO_2$ und X = OH, $OCH_3$ oder $OC_2H_5$,
g) y = $CH_3$, $(Z)_n$ = 2-Cl, 4-$NO_2$—2—$CH_3$ oder 4-$NO_2$—2—$OCH_3$ und X = $OC_2H_5$,
h) y = $C_2H_5$, $(Z)_n$ = H und X = OH
i) y = n-$C_3H_7$ oder i-$C_3H_7$, $(Z)_n$ = H und X = OH, $OCH_3$ oder $OC_2H_5$
k) x = $CH_3$, y = H oder $C_2H_5$ und $(Z)_n$ = H, 4-Cl oder 4-$OC_2H_5$ oder
l) x = $CH_3$, y = $CH_3$ und $(Z)_n$ = H, 4-Cl, 4-$OCH_3$, 4-$OC_2H_5$ oder 4-$NO_2$

bedeuten, ausgenommen sind, dadurch gekennzeichnet, daß man

a) eine Verbindung der Formel II

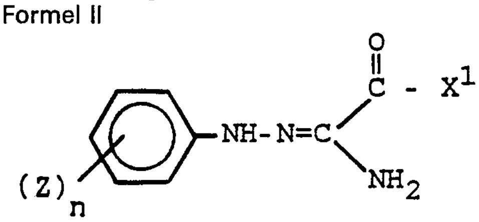

(II),

worin $X^1$ die Bedeutung von X außer Hydroxy besitzt,

$a_1$) mit einer Verbindung der Formel Y—CO—Cl oder
$a_2$) mit einem Säureanhydrid der Formel Y—CO—O—CO—Y oder
$a_3$) mit einem Orthoester der Formel Y—C$(OR^1)_3$, worin $R^1$ = $(C_1—C_4)$Alkyl bedeutet, oder
b) für Verbindungen mit Y = H, $(C_1—C_4)$Alkyl eine Verbindung der Formel III

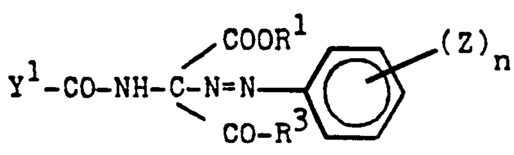

(III),

worin $Y^1$ = H oder $(C_1—C_4)$Alkyl und $R^3$ = $(C_1—C_4)$Alkoxy oder $CH_3$ bedeuten, mit einer Base umsetzt, wobei die unter $a_1$) und $a_2$) erhaltenen Verbindungen gegebenenfalls in Essigsäure erhitzt werden, und die erhaltenen Verbindungen der Formel I gegebenenfalls durch Derivatisierung in andere Verbindungen der Formel I oder deren Salze überführt werden.

9. Verfahren gemäß Anspruch 8, dadurch gekennzeichnet, daß in Formel I Y = $CCl_3$, $CHCl_2$; X = $(C_1—C_4)$-Alkoxy, OH; $(Z)_n$ = Cl, F, $OCH_3$ und n die Zahl 1, 2 oder 3 bedeuten.

10. Verfahren gemäß Anspruch 8, dadurch gekennzeichnet, daß in Formel I Y = $CCl_3$; X = $(C_1—C_4)$-Alkoxy und $(Z)_n$ = 2,4-$Cl_2$ bedeuten.

11. Verfahren gemäß Anspruch 8, dadurch gekennzeichnet, daß in Formel I Y = $CCl_3$; X = $OC_2H_5$ und $(Z)_n$ = 2,4-$Cl_2$ bedeuten.

**Revendications pour les Etats contractants: BE CH DE FR GB IT LI NL**

1. Produits phytosanitaires caractérisés en ce qu'ils contiennent un composé répondant à la formule I:

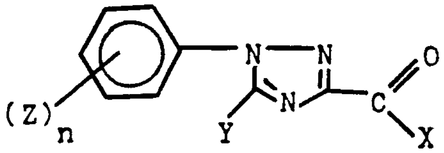 (I),

dans laquelle

les Z représentent chacun, indépendamment les uns des autres, un halogène, un nitro, un cyano, un trifluorométhyle, un alkyle en $C_1$—$C_4$, un alcoxy en $C_1$—$C_4$, un alkylthio en $C_1$—$C_4$, — les radicaux alkyles, alcoxy et alkylthio pouvant porter un ou plusieurs atomes d'halogènes, plus particulièrement de fluor ou de chlore —, un cycloalkyle en $C_3$—$C_6$ éventuellement porteur d'un alkyle en $C_1$—$C_4$, un phényle ou un phénoxy, les radicaux phényles et phénoxy pouvant porter un ou plusieurs atomes d'halogènes et/ou un trifluorométhyle,

Y représente l'hydrogène, un alkyle en $C_1$—$C_4$ qui peut être perhalogéné ou partiellement halogéné et/ou être porteur d'un seul alcoxy en $C_1$—$C_4$ ou alkylthio en $C_1$—$C_4$, un alcényle en $C_2$—$C_6$, un alcynyle en $C_2$—$C_6$, ou un cycloalkyle en $C_3$—$C_6$ éventuellement porteur d'un alkyle en $C_1$—$C_4$ et/ou d'un dichlorovinyle,

X représente un hydroxy, n alkyle en $C_1$—$C_4$, un cycloalcoxy en $C_2$—$C_6$, un phénylalcoxy à alcoxy en $C_1$—$C_6$, un phénoxy, un alcényloxy en $C_2$—$C_6$, un alcynyloxy en $C_2$—$C_6$, un alcoxy en $C_1$—$C_6$, un alkylthio en $C_1$—$C_6$ — les radicaux alcoxy et alkylthio pouvant porter un alcoxy en $C_1$ ou $C_2$, un mono- ou di-($C_1$—$C_4$)alkyl-aminocarbonyle, un phénylaminocarbonyle, un N-($C_1$—$C_4$)alkyl-phényl-aminocarbonyle, un mono- ou di-($C_1$—$C_6$)alkylamino, un alkylcarbonyloxy à alkyle en $C_1$—$C_6$, un alkylthio en $C_1$ ou $C_2$, un cyano ou un halogène —, un radical répondant à l'une des formules suivantes:

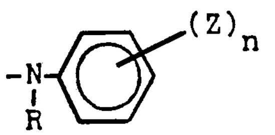 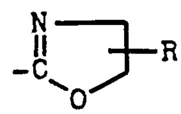

(dans lesquelles R représente l'hydrogène ou un alkyle en $C_1$—$C_4$), un mono- ou di-($C_1$—$C_4$)-alkylamino, un cycloalkylamino en $C_5$ ou $C_6$, un pipéridino, un morpholino, un diméthyl-2,6 morpholino ou un radical de formule:

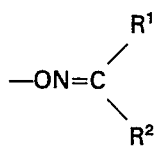

(dans laquelle $R^1$ et $R^2$ représentent chacun, indépendamment l'un de l'autre, un alkyle en $C_1$—$C_4$ et peuvent aussi former ensemble un radical cycloalkyle à 5, à 6 ou à 7 maillons) et

n représente un nombre égale à 0, à 1, à 2 ou à 3, ou, lorsque X représente un radical —OH, des sels d'un tel composé utilisables en agriculture.

2. Produits selon la revendication 1 caractérisés en ce qu'ils contiennent un composé de formule I selon la revendication 1 dans lequel:

Y représente un alkyle en $C_1$ ou $C_2$ dont les hydrogènes peuvent être remplacés, partiellement ou totalement, par F, Cl ou Br,

les Z représentent chacun, indépendamment les uns des autres un halogène, un alcoxy en $C_1$—$C_4$, un alkyle en $C_1$—$C_4$ ou —$CF_3$,

X représente un alcoxy en $C_1$—$C_6$ ou un hydroxy et

n est égal à 1, à 2 ou à 3.

3. Produits selon la revendication 2 caractérisés en ce que Y représente un radical $CCl_3$, $CHCl_2$, $CHF_2CF_2$ ou $CH_3$.

4. Produits selon l'une quelconque des revendications 1 à 3, caractérisés en ce qu'ils contiennent, en association avec un composé de formule I selon l'une quelconque des revendications 1 à 3, un herbicide pris dans l'ensemble constitué par les thiocarbamates, les carbamates, les halogeno-acétanilides, les esters d'acides phénoxy-, naphtoxy- et phénoxyphénoxy-carboxyliques, les dérivés de la dimédonoxime et les esters d'acides hétéroaryloxy-phénoxy-carboxyliques, ces derniers étant choisis parmi les esters d'acides quinolyloxy-, quinoxalyloxy-, pyridyloxy-, benzoxazolyloxy- et benzothiazolyloxy-phénoxy-carboxyliques.

5. Procédé pour protéger des plantes cultivées contre des effets secondaires phytotoxiques d'herbicides, procédé caractérisé en ce qu'on traite les plantes, les semences de plantes ou les surfaces de culture par une quantité efficace d'un composé de formule I selon l'une quelconque des revendications 1, 2 et 3, avant, aprés ou en même temps que le traitement par l'herbicide.

6. Procédé selon la revendication 5 caractérisé en ce qu'on utilise, comme herbicide, un thiocarbamate, un carbamate, un halogéno-acétanilide, un ester d'acide phénoxy-, naphtoxy- ou phénoxyphénoxy-carboxylique, un dérivé de la dimédon-oxime ou un ester d'acide hétéroaryloxy-phénoxy-carboxylique, ce dernier étant choisi parmi les esters d'acides quinolyloxy-, quinoxalyloxy-, pyridyloxy-, benzoxazolyloxy-, et benzothiazolyloxy-phénoxy-carboxyliques.

7. Application de composés de formule I selon l'une des revendications 1 et 2 à la protection de plantes cultivées contre des effects secondaires phytotoxiques d'herbicides.

8. Composés répondant à la formule I définie à la revendication 1, 2 ou 3, sauf ceux dans lesquels:

a) y = H, $(Z)_n$ = H et X = OH, $OCH_3$, $OC_2H_5$, ou $NHC_6H_5$,
b) y = H, $(Z)_n$ = 4—$OCH_3$ et X = OH, $OCH_3$ ou $NHC_6H_5$,
c) y = H, $(Z)_n$ = 3—$OCH_3$, 4—Cl ou 4—$CH_3$ et X = $OCH_3$,
d) y = H, $(Z)_n$ = 3—Cl et X = OH,
e) y = $CH_3$, $(Z)_n$ = H ou $OCH_3$ et X = OH,
f) y = $CH_3$, $(Z)_n$ = 4—$NO_2$ et X = OH, $OCH_3$ ou $OC_2H_5$,
g) y = $CH_3$, $(Z)_n$ = 2—Cl, 4—$NO_2$—2—$CH_3$ ou 4—$NO_2$—2—$OCH_3$ et X = $OC_2H_5$
h) y = $C_2H_5$, $(Z)_n$ = H et X = OH,
i) y = n—$C_3H_7$ ou i—$C_3H_7$, $(Z)_n$ = H et X = OH, $OCH_3$ ou $OC_2H_5$,
k) X = $CH_3$, y = H ou $C_2H_5$ et $(Z)_n$ = H, 4—Cl ou 4—$OC_2H_5$, ou
l) X = $CH_3$, y = $CH_3$ et $(Z)_n$ = H, 4—Cl, 4—$OCH_3$, 4—$OC_2H_5$ ou 4—$NO_2$.

9. Procédé pour préparer un composé selon la revendication 8, procédé caractérisé en ce que:

a) on fait réagir un composé répondant à la formule II:

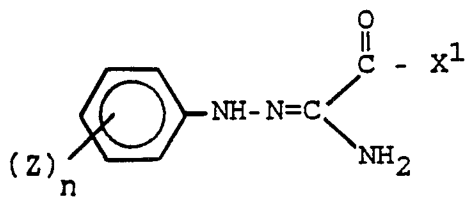
(II),

dans laquelle $X^1$ a la signification de X mais sans pouvoir représenter un hydroxy,

a1) avec un composé de formule Y—CO—Cl ou
a2) avec un anhydride d'acide de formule Y—CO—O—CO—Y ou
a3) avec un ortho-ester de formule Y—$C(OR^1)_3$ dans lequel $R^1$ represente un alkyle en $C_1$—$C_4$, ou

b) dans le cas de composés dans lesquels Y represente H ou un alkyle en $C_1$—$C_4$ on fait réagir avec un base un composé répondant à la formule III:

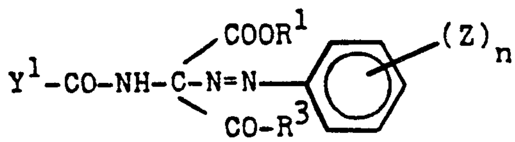
(III),

dans laquelle $Y^1$ représente H ou un alkyle en $C_1$—$C_4$ et $R^3$ représente un alcoxy en $C_1$—$C_4$ ou $CH_3$, on chauffe éventuellement dans l'acide acétique les composés obtenus sous a1) et a2), et on transforme éventuellement les composés de formule I obtenus, par dérivation, en d'autres composés de formule I ou en leurs sels.

10. Composés de formule I selon la revendication 8 dans lesquels Y représente $CCl_3$ ou $CHCl_2$, X représente un alcoxy en $C_1$—$C_4$ ou un radical OH, Z représente Cl, F ou $OCH_3$, et n désigne un nombre égal à 1, à 2 ou à 3.

11. Composés de formule I selon la revendication 8 dans lesquels Y représente $CCl_3$, X représente un alcoxy en $C_1$—$C_4$ et $(Z)_n$ représente deux atomes de chore, l'un en 2, l'autre en 4.

12. Composé de formule I selon la revendication 8 dans lequel Y représente $CCl_3$, X représente $OC_2H_5$ et $(Z)_n$ représente deux atomes de chlore, l'un en 2, l'autre en 4.

**Revendications pour l'Etat contractant: AT**

1. Produits phytosanitaires caractérisés en ce qu'ils contiennent un composé répondant à la formule I:

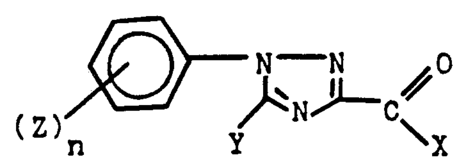
(I),

dans laquelle

les Z représentent chacun, indépendamment les uns des autres, un halogène, un nitro, un cyano, un trifluorométhyle, un alkyle en $C_1$—$C_4$, un alcoxy en $C_1$—$C_4$, un alkylthio en $C_1$—$C_4$, — les radicaux alkyle, alcoxy et alkylthio pouvant porter un ou plusieurs atomes d'halogènes, plus particulièrement de fluor ou de chlore —, un cycloalkyle en $C_3$—$C_6$ éventuellement porteur d'un alkyle en $C_1$—$C_4$, un phényle ou un phénoxy, les radicaux phényles et phénoxy pouvant porter un ou plusieurs atomes d'halogènes et/ou un trifluorométhyle,

Y représente l'hydrogène, un alkyle en $C_1$—$C_4$ qui peut être perhalogéné ou partiellement halogéné et/ou être porteur d'un seul alcoxy en $C_1$—$C_4$ ou alkylthio en $C_1$—$C_4$, un alcényle en $C_2$—$C_6$, un alcynyle en $C_2$—$C_6$, ou un cycloalkyle en $C_3$—$C_6$ éventuellement porteur d'un alkyle en $C_1$—$C_4$ et/ou d'un dichlorovinyle,

X représente un hydroxy, un alkyle en $C_1$—$C_4$, un cycloalcoxy en $C_3$—$C_6$, un phénylalcoxy à alcoxy en $C_1$—$C_6$, un phénoxy, un alcényloxy en $C_2$—$C_6$, un alcynyloxy en $C_2$—$C_6$, un alcoxy en $C_1$—$C_6$, un alkylthio en $C_1$—$C_6$ — les radicaux alcoxy et alkylthio pouvant porter un alcoxy en $C_1$ ou $C_2$, un mono- ou di-($C_1$—$C_4$)alkyl-aminocarbonyle, un phénylaminocarbonyle, un N-($C_1$—$C_4$)alkyl-phényl-aminocarbonyle, un mono- ou di-($C_1$—$C_6$)alkylamino, un alkylcarbonyloxy à alkyle en $C_1$—$C_6$, un alkylthio en $C_1$ ou $C_2$, un cyano ou un halogène —, un radical répondant à l'une des formules suivantes:

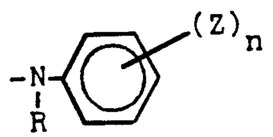

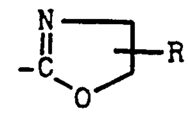

(dans lesquelles R représente l'hydrogène ou un alkyle en $C_1$—$C_4$), un mono- ou di-($C_1$—$C_4$)-alkylamino, un cycloalkylamino en $C_5$ ou $C_6$, un pipéridino, un morpholino, un diméthyl-2,6 morpholino ou un radical de formule:

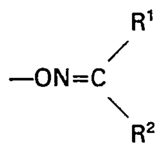

(dans laquelle $R^1$ et $R^2$ représentent chacun, indépendamment l'un de l'autre, un alkyle en $C_1$—$C_4$ et peuvent aussi former ensemble un radical cycloalkyle à 5, à 6 ou à 7 maillons) et

n représente un nombre égal à 0, à 1, à 2, à 3, ou, lorsque X représente un radical —OH, des sels d'un tel composé utilisables en agriculture.

2. Produits selon la revendication 1 caractérisés en ce qu'ils contiennent un composé de formule I selon la revendication 1 dans lequel:

Y représente un alkyle en $C_1$ ou $C_2$ dont les hydrogènes peuvent être remplacés, partiellement ou totalement, par F, Cl ou Br,

les Z représentent chacun, indépendamment les uns des autres un halogène, un alcoxy en $C_1$—$C_4$, un alkyle en $C_1$—$C_4$ ou —$CF_3$,

X représente un alcoxy en $C_1$—$C_6$ ou un hydroxy et

n est égal à 1, à 2, ou à 3.

3. Produits selon la revendication 2 caractérisés en ce que Y représente un radical $CCl_3$, $CHCl_2$, $CHF_2CF_2$ ou $CH_3$.

4. Produits selon l'une quelconque des revendications 1 à 3, caractérisés en ce qu'ils contiennent, en association avec un composé de formule I selon l'une quelconque des revendications 1 à 3, un herbicide pris dans l'ensemble constitué par les thiocarbamates, les carbamates, les halogeno-acétanilides, les esters d'acides phénoxy-, naphthoxy- et phénoxyphénoxy-carboxyliques, les dérivés de la dimédonoxime et les esters d'acides hétéroaryloxy-phénoxy-carboxyliques, ces derniers étant choisis parmi les esters d'acides quinolyloxy-, quinoxalyloxy-, pyridyloxy-, benzoxazolyloxy- et benzothiazolyloxy-phénoxy-carboxyliques.

5. Procédé pour protéger des plantes cultivées contre des effets secondaires phytotoxiques d'herbicides, procédé caractérisé en ce qu'on traite les plantes, les semences de plantes ou les surfaces de culture par une quantité efficace d'un composé de formule I selon l'une quelconque des revendications 1, 2 et 3, avant, aprés ou en même temps que le traitement par l'herbicide.

6. Procédé selon la revendication 5 caractérisé en ce qu'on utilise, comme herbicide, un thiocarbamate, un carbamate, un halogéno-acétanilide, un ester d'acide phénoxy-, naphtoxy- ou phénoxyphénoxy-carboxylique, un dérivé de la dimédon-oxime ou un ester d'acide hétéroaryloxy-phénoxy-carboxylique, ce dernier etant choisi parmi les ester d'acide quinolyloxy-, quinoxalyloxy-, pyridyloxy-, benzoxazolyloxy-, et benzothiazolyloxy-phénoxy-carboxyliques.

7. Application de composés de formule I selon l'une des revendications 1 et 2 à la protection de plantes cultivées contre des effects secondaires phytotoxiques d'herbicides.

8. Procédé pour préparer des composés répondant à la formule I définie à la revendication 1, 2 ou 3, à l'exception de ceux dans lesquels:

a) y = H, $(Z)_n$ = H et X = OH, $OCH_3$, $OC_2H_5$, ou $NHC_6H_5$,

b) y = H, $(Z)_n$ = 4—$OCH_3$ et X = OH, $OCH_3$ ou $NHC_6H_5$,
c) y = H, $(Z)_n$ = 3—$OCH_3$, 4—Cl ou 4—$CH_3$ et X = $OCH_3$,
d) y = H, $(Z)_n$ = 3—Cl et X = OH,
e) y = $CH_3$, $(Z)_n$ = H ou $OCH_3$ et X = OH,
f) y = $CH_3$, $(Z)_n$ = 4—$NO_2$ et X = OH, $OCH_3$ ou $OC_2H_5$,
g) y = $CH_3$, $(Z)_n$ = 2—Cl, 4—$NO_2$—2—$CH_3$ ou 4—$NO_2$—2—$OCH_3$ et X = $OC_2H_5$
h) y = $C_2H_5$, $(Z)_n$ = H et X = OH,
i) y = n—$C_3H_7$ ou i—$C_3H_7$, $(Z)_n$ = H et X = OH, $OCH_3$ ou $OC_2H_5$,
k) X = $CH_3$, y = H ou $C_2H_5$ et $(Z)_n$ = H, 4—Cl ou 4—$OC_2H_5$, ou
l) X = $CH_3$, y = $CH_3$ et $(Z)_n$ = H, 4—Cl, 4—$OCH_3$, 4—$OC_2H_5$ ou 4—$NO_2$,

procédé caractérisé en ce que:

a) on fait réagir un composé répondant à la formule II:

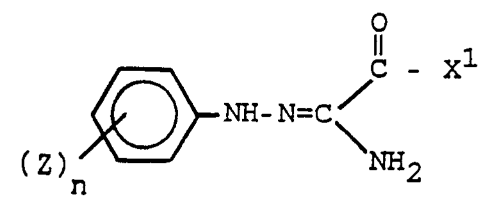

(II),

dans laquelle $X^1$ a la signification de X mais sans pouvoir représenter un hydroxy,

a1) avec un composé de formule Y—CO—Cl ou

a2) avec un anhydride d'acide de formule Y—CO—O—CO—Y ou

a3) avec un ortho-ester de formule Y—$C(OR^1)_3$ dans lequel $R^1$ représente un alkyle en $C_1$—$C_4$, ou

b) dans le cas de composés dans lesquels Y représente H ou un alkyle en $C_1$—$C_4$ on fait réagir avec une base un composé répondant à la formule III:

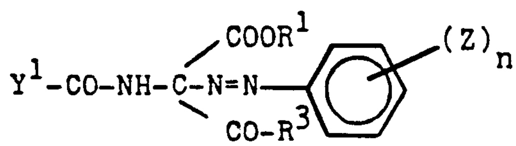

(III),

dans laquelle $Y^1$ représente H ou un alkyle en $C_1$—$C_4$ et $R^3$ représente un alcoxy en $C_1$—$C_4$ ou $CH_3$, on chauffe éventuellement dans l'acide acétique les composés obtenus sous a1) et a2), et on transforme éventuellement les composés de formule I obtenus, par dérivation, en d'autres composés de formule I ou en leurs sels.

9. Procédé selon la revendication 8, caractérisé en ce que, dans la formule I, Y représente $CCl_3$ ou $CHCl_2$, X représente un alcoxy en $C_1$—$C_4$ ou un radical OH, Z représente Cl, F ou $OCH_3$, ent n désigne un nombre égal à 1, à 2 ou à 3.

10. Procédé selon la revendication 8, caractérisé en ce que, dans la formule I, Y représente $CCl_3$, X représente un alcoxy en $C_1$—$C_4$ et $(Z)_n$ représente deux atomes de chore, l'un en 2, l'autre en 4.

11. Procédé selon la revendication 8, caractérisé en ce que, dans la formule I, Y représente $CCl_3$, X représente $OC_2H_5$ et $(Z)_n$ représente deux atomes de chlore, l'un en 2, l'autre en 4.

**Claims for the Contracting States: BE CH DE FR GB IT LI NL**

1. A plant protection agent which contains a compound of the formula I

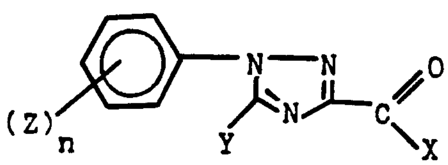

(I),

in which

the Zs are identical or different and denote halogen, nitro, cyano, trifluoromethyl, ($C_1$—$C_4$)-alkyl, ($C_1$—$C_4$)-alkoxy or ($C_1$—$C_4$)-alkylthio, it being possible for the alkyl, alkoxy and alkylthio groups to be substituted by one or more halogen atoms, in particular fluorine or chlorine, or ($C_3$—$C_6$)-cycloalkyl which can be substituted by ($C_1$—$C_4$)-alkyl, or denotes phenyl or phenoxy, it being possible for phenyl and phenoxy to be monosubstituted or polysubstituted by halogen and/or monosubstituted by trifluoromethyl,

Y denotes hydrogen, ($C_1$—$C_4$)-alkyl which can be wholly or partially substituted by halogen atoms and/or monosubstituted by ($C_1$—$C_4$)-alkoxy or ($C_1$—$C_4$)-alkylthio, or ($C_2$—$C_6$)-alkenyl or ($C_2$—$C_6$)-alkinyl, or ($C_3$—$C_6$)-cycloalkyl which can be substituted by ($C_1$—$C_4$)-alkyl and/or a dichlorovinyl radical, and

X denotes hydroxyl, ($C_1$—$C_4$)-alkyl, ($C_3$—$C_6$)-cycloalkoxy, phenyl-($C_1$—$C_6$)-alkoxy, phenoxy, ($C_2$—$C_6$)-alkenyloxy, ($C_2$—$C_6$)-alkinyloxy or ($C_1$—$C_6$)-alkoxy or ($C_1$—$C_6$)-alkylthio, it being possible for the alkoxy or alkylthio group to be substituted by ($C_1$—$C_2$)-alkoxy, mono-($C_1$—$C_4$)-alkylaminocarbonyl, di-($C_1$—$C_4$)-alkylaminocarbonyl, phenylaminocarbonyl, N-($C_1$—$C_4$)-alkylphenylaminocarbonyl, mono-($C_1$—$C_6$)-alkylamino, di-($C_1$—$C_6$)-alkylamino, ($C_1$—$C_6$)-alkylcarbonyloxy, ($C_1$—$C_2$)-alkylthio, cyano or halogen, or denotes a radical of the formulae

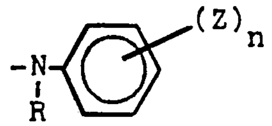 or 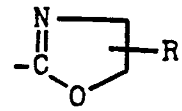 ,

in which R in each case denotes hydrogen or ($C_1$—$C_4$)-alkyl, or denotes mono-($C_1$—$C_4$)-alkylamino, di-($C_1$—$C_4$)-alkylamino, ($C_5$—$C_6$)-cycloalkylamino, piperidino, morpholino or 2,6-dimethylmorpholino or a radical of the formula

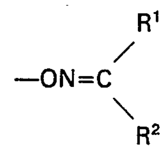

in which $R^1$ and $R^2$ can be identical or different and denote ($C_1$—$C_4$)-alkyl radicals, and in which $R^1$ and $R^2$ together can also form a 5-membered, 6-membered or 7-membered cycloalkyl radical, and

n denotes the number 0, 1, 2, or 3, or, in the event that X = OH, salts thereof can be employed for agriculture.

2. An agent as claimed in claim 1, which contains a compound of the formula I of claim 1 in which
Y denotes ($C_1$—$C_2$)-alkyl which can be wholly or partially substituted by F, Cl or Br,
the Zs are identical or different and denote halogen, ($C_1$—$C_4$)-alkoxy, ($C_1$—$C_4$)-alkyl or $CF_3$,
X denotes ($C_1$—$C_6$)-alkoxy or hydroxyl and n denotes 1, 2 or 3.

3. An agent as claimed in claim 2, wherein Y denotes $CCl_3$, $CHCl_2$, $CHF_2CF_2$ or $CH_3$.

4. An agent as claimed in any of claims 1, 2 or 3, which, in combination with a compound of the formula I of claims 1, 2 or 3, additionally contains a herbicide from the group comprising thiolcarbamates, carbamates, halogenoacetanilides, phenoxy-, naphthoxy- or phenoxyphenoxy- carboxylic acid esters, dimedone oxime derivatives or heteroaryloxyphenoxycarboxylic acid esters, the latter selected from the group comprising quinolyloxy-, quinoxalyloxy-, pyridyloxy-, benzoxazolyloxy- or benzothiazolyloxyphenoxy-carboxylic acid esters.

5. A process for protecting crop plants against the phytotoxic side-effects of herbicides, which comprises treating the plants, plant seeds or cultivated areas with an effective amount of a compound of the formula I of claims 1, 2 or 3, before, after or at the same time as the herbicide.

6. The process as claimed in claim 5, wherein the herbicide employed is a thiocarbamate, carbamate, halogenoacetanilide, phenoxy, naphthoxy- or phenoxyphenoxycarboxylic acid ester, a dimedone oxime derivative or a heteroaryloxyphenoxycarboxylic acid ester, the latter selected from the group comprising quinolyloxy-, quinoxalyloxy-, pyridyloxy-, benzoxazolyloxy- or benzothiazolyloxyphenoxy-carboxylic acid esters.

7. The use of a compound of the formula I of claims 1 or 2 for protecting crop plants against the phytotoxic side-effects of herbicides.

8. A compound of the formula I as defined in claim 1, 2 or 3, with the exception of those in which
a) y denotes H, $(Z)_n$ denotes H and X denotes OH, $OCH_3$, $OC_2H_5$ or $NHC_6H_5$,
b) y denotes H, $(Z)_n$ denotes 4—$OCH_3$ and X denotes OH, $OCH_3$ or $NHC_6H_5$,
c) y denotes H, $(Z)_n$ denotes 3—$OCH_3$, 4—Cl, 4—$CH_3$ and X denotes $OCH_3$,
d) y denotes H, $(Z)_n$ denotes 3—Cl and X denotes OH,
e) y denotes $CH_3$, $(Z)_n$ denotes H or $OCH_3$ and X denotes OH,
f) y denotes $CH_3$, $(Z)_n$ denotes 4—$NO_2$ and X denotes OH, $OCH_3$ or $OC_2H_5$,
g) y denotes $CH_3$, $(Z)_n$ denotes 2—Cl, 4—$NO_2$—2—$CH_3$ or 4—$NO_2$—2—$OCH_3$ and X denotes $OC_2H_5$,
h) y denotes $C_2H_5$, $(Z)_n$ denotes H and X denotes OH
i) y denotes n—$C_3H_7$ or i—$C_3H_7$, $(Z)_n$ denotes H and X denotes OH, $OCH_3$ or $OC_2H_5$
k) x denotes $CH_3$, y denotes H or $C_2H_5$ and $(Z)_n$ denotes H, 4—Cl or 4—$OC_2H_5$ or
l) x denotes $CH_3$, y denotes $CH_3$ and $(Z)_n$ denotes H, 4—Cl, 4—$OCH_3$, 4—$OC_2H_5$ or 4—$NO_2$.

9. A process for the preparation of a compound of claim 8, which comprises
a) reacting a compound of the formula II

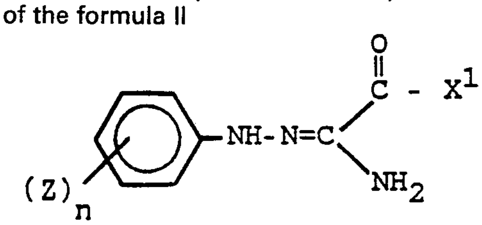 (II),

in which $X^1$ has the meaning of X with the exception of hydroxyl,

$a_1$) with a compound of the formula Y—CO—Cl or

$a_2$) with an acid anhydride of the formula Y—CO—O—CO—Y or

$a_3$) with an orthoester of the formula $Y—C(OR^1)_3$ in which $R^1$ denotes ($C_1$—$C_4$)-alkyl, or

b) in the case of a compound in which Y = H or ($C_1$—$C_4$)-alkyl, reacting a compound of the formula III

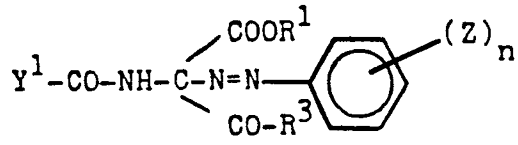

(III),

in which $Y^1$ denotes H or ($C_1$—$C_4$)-alkyl and $R^3$ denotes ($C_1$—$C_4$)-alkoxy or $CH_3$, with a base, the compounds obtained under $a_1$ and $a_2$) being heated, if appropriate, in acetic acid, and the resulting compounds of the formula I being converted, if appropriate, into other compounds of the formula I or salts thereof by the formation of derivatives.

10. A compound of the formula I as claimed in claim 8, in which Y denotes $CCl_3$ or $CHCl_2$, X denotes ($C_1$—$C_4$)-alkoxy or OH, Z denotes Cl, F or $OCH_3$ and n denotes the number 1, 2 or 3.

11. A compound of the formula I as claimed in claim 8, in which Y denotes $CCl_3$, X denotes ($C_1$—$C_4$)-alkoxy and $(Z)_n$ denotes 2,4—$Cl_2$.

12. A compound of the formula I as claimed in claim 8, in which Y denotes $CCl_3$, X denotes $OC_2H_5$ and $(Z)_n$ denotes 2,4—$Cl_2$.

**Claims for the Contracting State: AT**

1. A plant protection agent which contains a compound of the formula I

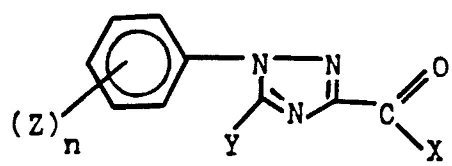

(I),

in which

the Zs are identical or different and denote halogen, nitro, cyano, trifluoromethyl, ($C_1$—$C_4$)-alkyl, ($C_1$—$C_4$)-alkoxy or ($C_1$—$C_4$)-alkylthio, it being possible for the alkyl, alkoxy and alkylthio groups to be substituted by one or more halogen atoms, in particular fluorine or chlorine, or ($C_3$—$C_6$)-cycloalkyl which can be substituted by ($C_1$—$C_4$)-alkyl, or denotes phenyl or phenoxy, it being possible for phenyl and phenoxy to be monosubstituted or polysubstituted by halogen and/or monosubstituted by trifluoromethyl,

Y denotes hydrogen, ($C_1$—$C_4$)-alkyl which can be wholly or partially substituted by halogen atoms and/or monosubstituted by ($C_1$—$C_4$)-alkoxy or ($C_1$—$C_4$)-alkylthio, or ($C_2$—$C_6$)-alkenyl or ($C_2$—$C_6$)-alkinyl, or ($C_3$—$C_6$)-cycloalkyl which can be substituted by ($C_1$—$C_4$)-alkyl and/or a dichlorovinyl radical, and

X denotes hydroxyl, ($C_1$—$C_4$)-alkyl, ($C_3$—$C_6$)-cycloalkoxy, phenyl-($C_1$—$C_6$)-alkoxy, phenoxy, ($C_2$—$C_6$)-alkenyloxy, ($C_2$—$C_6$)-alkinyloxy or ($C_1$—$C_6$)-alkoxy or ($C_1$—$C_6$)-alkylthio, it being possible for the alkoxy or alkylthio group to be substituted by ($C_1$—$C_2$)-alkoxy, mono-($C_1$—$C_4$)-alkylaminocarbonyl, di-($C_1$—$C_4$)-alkylaminocarbonyl, phenylaminocarbonyl, N-($C_1$—$C_4$)-alkylphenylaminocarbonyl, mono($C_1$—$C_6$)-alkylamino, di-($C_1$—$C_6$)-alkylamino, ($C_1$—$C_6$)-alkylcarbonyloxy, ($C_1$—$C_2$)-alkylthio, cyano or halogen, or denotes a radical of the formulae

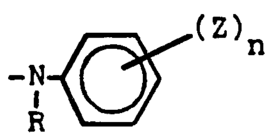

or

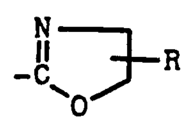

,

in which R in each case denotes hydrogen or ($C_1$—$C_4$)-alkyl, or denotes mono-($C_1$—$C_4$)-alkylamino, di-($C_1$—$C_4$)-alkylamino, ($C_5$—$C_6$)-cycloalkylamino, piperidino, morpholino or 2,6-dimethylmorpholino or a radical of the formula

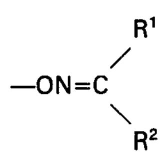

in which $R^1$ and $R^2$ can be identical or different and denote ($C_1$—$C_4$)-alkyl radicals, and in which $R^1$ and $R^2$ together can also form a 5-membered, 6-membered or 7-membered cycloalkyl radical, and

n denotes the number 0, 1, 2, or 3, or, in the event that X = OH, salts thereof can be employed for agriculture.

2. An agent as claimed in claim 1, which contains a compound of the formula I of claim 1 in which
Y denotes ($C_1$—$C_2$)-alkyl which can be wholly or partially substituted by F, Cl or Br,
the Zs are identical or different and denote halogen, ($C_1$—$C_4$)-alkoxy, ($C_1$—$C_4$)-alkyl or $CF_3$,
X denotes ($C_1$—$C_6$)-alkoxy or hydroxyl and n denotes 1, 2 or 3.

3. An agent as claimed in claim 2, wherein Y denotes $CCl_3$, $CHCl_2$, $CHF_2CF_2$ or $CH_3$.

4. An agent as claimed in any of claims 1, 2 or 3, which, in combination with a compound of the formula I of claims 1, 2 or 3, additionally contains a herbicide from the group comprising thiolcarbamates, carbamates, halogenoacetanilides, phenoxy-, naphthoxy-, phenoxyphenoxy- or carboxylic acid esters, dimedone oxime derivatives or heteroaryloxyphenoxycarboxylic acid esters, the latter selected from the group comprising quinolyloxy-, quinoxalyloxy-, pyridyloxy-, benzoxazolyloxy- or benzothiazolyloxyphenoxy-carboxylic acid esters.

5. A process for protecting crop plants against the phytotoxic side-effects of herbicides, which comprises treating the plants, plant seeds or cultivated areas with an effective amount of a compound of the formula I of claims 1, 2 or 3, before, after or at the same time as the herbicide.

6. The process as claimed in claim 5, wherein the herbicide employed is a thiolcarbamate, carbamate, halogenoacetanilide, phenoxy-, naphthoxy- or phenoxyphenoxycarboxylic acid ester, a dimedone oxime derivative or a heteroaryloxyphenoxycarboxylic acid ester, the latter selected from the group comprising quinolyloxy-, quinoxalyloxy-, pyridyloxy-, benzoxazolyloxy- or benzothiazolyloxyphenoxy-carboxylic acid esters.

7. The use of a compound of the formula I of claims 1 or 2 for protecting crop plants against the phytotoxic side-effects of herbicides.

8. A process for preparing a compound of the formula I as defined in claim 1, 2 or 3, with the exception of those in which
a) y denotes H, $(Z)_n$ denotes H and X denotes OH, $OCH_3$, $OC_2H_5$ or $NHC_6H_5$,
b) y denotes H, $(Z)_n$ denotes 4—$OCH_3$ and X denotes OH, $OCH_3$ or $NHC_6H_5$,
c) y denotes H, $(Z)_n$ denotes 3—$OCH_3$, 4—Cl, 4—$CH_3$ and X denotes $OCH_3$,
d) y denotes H, $(Z)_n$ denotes 3—Cl and X denotes OH,
e) y denotes $CH_3$, $(Z)_n$ denotes H or $OCH_3$ and X denotes OH,
f) y denotes $CH_3$, $(Z)_n$ denotes 4—$NO_2$ and X denotes OH, $OCH_3$ or $OC_2H_5$,
g) y denotes $CH_3$, $(Z)_n$ denotes 2—Cl, 4—$NO_2$—2—$CH_3$ or 4—$NO_2$—2—$OCH_3$ and X denotes $OC_2H_5$,
h) y denotes $C_2H_5$, $(Z)_n$ denotes H and X denotes OH
i) y denotes n—$C_3H_7$ or i—$C_3H_7$, $(Z)_n$ denotes H and X denotes OH, $OCH_3$ or $OC_2H_5$
k) x denotes $CH_3$, y denotes H or $C_2H_5$ and $(Z)_n$ denotes H, 4—Cl or 4—$OC_2H_5$ or
l) x denotes $CH_3$, y denotes $CH_3$ and $(Z)_n$ denotes H, 4—Cl, 4—$OCH_3$, 4—$OC_2H_5$ or 4—$NO_2$.
which comprises
a) reacting a compound of the formula II

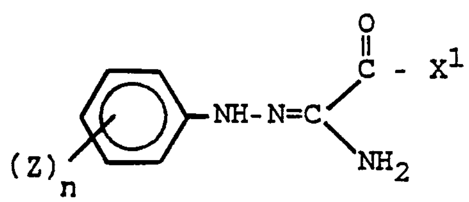

(II),

in which $X^1$ has the meaning of X with the exception of hydroxyl,
$a_1$) with a compound of the formula Y—CO—Cl or
$a_2$) with an acid anhydride of the formula Y—CO—O—CO—Y or
$a_3$) with an orthoester of the formula Y—C$(OR^1)_3$ in which $R^1$ denotes ($C_1$—$C_4$)-alkyl, or
b) in the case of a compound in which Y = H or ($C_1$—$C_4$)-alkyl, reacting a compound of the formula III

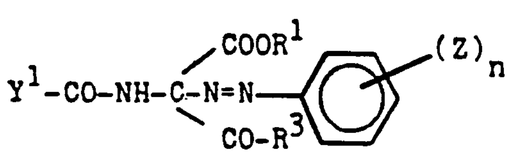

(III),

in which $Y^1$ denotes H or ($C_1$—$C_4$)-alkyl and $R^3$ denotes ($C_1$—$C_4$)-alkoxy or $CH_3$, with a base, the compounds obtained under $a_1$ and $a_2$) being heated, if appropriate, in acetic acid, and the resulting compounds of the formula I being converted into, if appropriate, other compounds of the formula I or salts thereof by the formation of derivatives.

9. The process as claimed in claim 8, wherein, in formula I, Y denotes $CCl_3$ or $CHCl_2$, X denotes ($C_1$—$C_4$)-alkoxy or OH, $(Z)_n$ denotes Cl, F or $OCH_3$ and n denotes the number 1, 2 or 3.

10. The process as claimed in claim 8, wherein, in formula I, Y denotes $CCl_3$, X denotes $(C_1—C_4)$-alkoxy and $(Z)_n$) denotes 2,4—$Cl_2$.

11. The process as claimed in claim 8, wherein, in formula I, Y denotes $CCl_3$, X denotes $OC_2H_5$ and $(Z)_n$ denotes 2,4—$Cl_2$.